(19) Europäisches Patentamt / European Patent Office / Office européen des brevets

(11) **EP 4 483 999 A1**

(12) **EUROPEAN PATENT APPLICATION**
published in accordance with Art. 153(4) EPC

(43) Date of publication:
**01.01.2025 Bulletin 2025/01**

(21) Application number: **23760095.2**

(22) Date of filing: **24.02.2023**

(51) International Patent Classification (IPC):
**B01J 19/00** (2006.01)   **C12M 1/00** (2006.01)
**C12M 1/42** (2006.01)   **C12N 1/20** (2006.01)

(52) Cooperative Patent Classification (CPC):
**B01J 19/00; C12M 1/00; C12M 1/42; C12N 1/20**

(86) International application number:
**PCT/JP2023/006701**

(87) International publication number:
**WO 2023/163096 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **24.02.2022 JP 2022026834**

(71) Applicant: **University Public Corporation Osaka Osaka City 545-0051 (JP)**

(72) Inventors:
• IIDA, Takuya
  Sakai-shi, Osaka 599-8531 (JP)
• TOKONAMI, Shiho
  Sakai-shi, Osaka 599-8531 (JP)
• HAYASHI, Kota
  Sakai-shi, Osaka 599-8531 (JP)
• FUJIWARA, Masazumi
  Osaka-shi, Osaka 558-8585 (JP)

(74) Representative: **Bals & Vogel Patentanwälte PartGmbB**
**Konrad-Zuse-Str. 4**
**44801 Bochum (DE)**

(54) **MICRO-OBJECT CONCENTRATION METHOD, MICRO-OBJECT CONCENTRATION KIT, AND MICRO-OBJECT CONCENTRATION SYSTEM**

(57)    A microscopic object condensation method includes first to third steps. The first step is a step of preparing an optical fiber (50) including a tip end provided with a metallic thin film (52). The second step is a step of arranging the tip end of the optical fiber (50) in liquid where a plurality of microscopic objects are dispersed. The third step is a step of producing convection by heating of liquid around the tip end of the optical fiber (50) by introduction of light at a wavelength included in a range of an absorption wavelength of the metallic thin film (52) into the optical fiber (50).

**EP 4 483 999 A1**

FIG.4

```
                    ┌──────────────┐
                    │    START     │
                    └──────┬───────┘
                           │        ⌒S1
        ┌──────────────────▼──────────────────┐
        │        PREPARE OPTICAL FIBER         │
        └──────────────────┬──────────────────┘
                           │        ⌒S2
        ┌──────────────────▼──────────────────┐
        │ PREPARE SAMPLE IN WHICH MICROSCOPIC  │
        │       OBJECTS ARE DISPERSED          │
        └──────────────────┬──────────────────┘
                           │        ⌒S3
        ┌──────────────────▼──────────────────┐
        │         INTRODUCE SURFACTANT         │
        └──────────────────┬──────────────────┘
                           │        ⌒S4
        ┌──────────────────▼──────────────────┐
        │    SET CONDENSATION KIT ON SAMPLE    │
        │       STAGE AND DROP SAMPLE          │
        └──────────────────┬──────────────────┘
                           │        ⌒S5
        ┌──────────────────▼──────────────────┐
        │       START IMAGING OF SAMPLE        │
        └──────────────────┬──────────────────┘
                           │        ⌒S6
        ┌──────────────────▼──────────────────┐
        │     ADJUST POSITION AND HEIGHT OF    │
        │            SAMPLE STAGE              │
        └──────────────────┬──────────────────┘
                           │        ⌒S7
        ┌──────────────────▼──────────────────┐
        │ ADJUST POSITION AND HEIGHT OF FIBER  │
        │            END IN SAMPLE             │
        └──────────────────┬──────────────────┘
                           │        ⌒S8
        ┌──────────────────▼──────────────────┐
        │           EMIT LASER BEAMS           │
        └──────────────────┬──────────────────┘
                           │        ⌒S9
        ┌──────────────────▼──────────────────┐
        │          CONDENSATION STEP           │
        └──────────────────┬──────────────────┘
                           │        ⌒S10
        ┌──────────────────▼──────────────────┐
        │     STOP EMISSION OF LASER BEAMS     │
        └──────────────────┬──────────────────┘
                           │        ⌒S11
        ┌──────────────────▼──────────────────┐
        │        QUIT IMAGING OF SAMPLE        │
        └──────────────────┬──────────────────┘
                           │        ⌒S12
        ┌──────────────────▼──────────────────┐
        │  CALCULATE THE NUMBER OF CONDENSED   │
        │        MICROSCOPIC OBJECTS           │
        └──────────────────┬──────────────────┘
                           │
                    ┌──────▼───────┐
                    │     END      │
                    └──────────────┘
```

**Description**

TECHNICAL FIELD

The present disclosure relates to a microscopic object condensation method, a microscopic object condensation kit, and a microscopic object condensation system, and more particularly to a technique for condensation of a plurality of microscopic objects dispersed in liquid.

BACKGROUND ART

WO2018/159706 (PTL 1), WO2020/218347 (PTL 2), and the like disclose a technique that enables highly efficient condensation of a plurality of microscopic objects dispersed in liquid.

CITATION LIST

PATENT LITERATURE

PTL 1: WO2018/159706
PTL 2: WO2020/218347

SUMMARY OF INVENTION

TECHNICAL PROBLEM

In the inventions disclosed in PTLs 1 and 2, a photothermal conversion region is provided at a bottom surface of a container, a main surface of a substrate, and the like. In this case, a plurality of microscopic objects dispersed in liquid are condensed in the vicinity of the photothermal conversion region, which means that a location where microscopic objects can be condensed is limited to the bottom surface of the container, the main surface of the substrate, and the like. In development of the technique for condensation of microscopic objects in various fields in the future, desirably, microscopic objects can be condensed at any location in liquid.

The present disclosure was made to solve the problem above, and one of objects of the present disclosure is to condense microscopic objects at any location in liquid.

SOLUTION TO PROBLEM

(1) A microscopic object condensation method according to a first aspect of the present disclosure includes first to third steps. The first step is a step of preparing an optical fiber including a tip end provided with a photothermal conversion material. The second step is a step of arranging the tip end in liquid where a plurality of microscopic objects are dispersed. The third step is a step of introducing light into the optical fiber to heat the liquid around the tip end thereby producing convection, the light having a wavelength included in a range of an absorption wavelength of the photothermal conversion material.

(2) The arranging the tip end (second step) includes adjusting a position or a height of the tip end of the optical fiber in the liquid.

(3) The preparing the optical fiber (first step) includes pre-treating the photothermal conversion material to stabilize a transmittance ratio or an extinction ratio of the photothermal conversion material with respect to variation in output of light that propagates through the optical fiber.

(4) The producing convection (third step) includes generating a microbubble at the tip end of the optical fiber and condensing the plurality of microscopic objects in a region between the tip end and the microbubble.

(5) The arranging the tip end includes setting arrangement of the optical fiber with respect to a substrate where the liquid is held to one of contactless arrangement and contact arrangement. The contactless arrangement refers to arrangement where a propagation path of the optical fiber is not in contact with the substrate. The contact arrangement refers to arrangement where the propagation path of the optical fiber is in contact with the substrate.

(6) The arranging the tip end is setting the optical fiber in the contactless arrangement. The producing convection includes condensing the plurality of microscopic objects at the tip end without generation of a microbubble at the pre-treated tip end.

(7) The arranging the tip end is setting the optical fiber in the contact arrangement. The producing convection includes condensing the plurality of microscopic objects along an optical path of light emitted from the tip end.

(8) The microscopic object condensation method further includes introducing a surfactant into the liquid prior to the producing convection (third step).

(9) The introducing the surfactant includes preparing a concentration of the surfactant in the liquid to a critical micelle concentration.

(10) Each of the plurality of microscopic objects is a quantum sensor. The quantum sensor includes at least one of nanodiamond, a fluorescent molecule, a quantum dot, a metallic nanoparticle, and a metallic nanorod.

(11) A microscopic object condensation method according to a second aspect of the present disclosure condenses a plurality of microscopic objects dispersed in liquid. The microscopic object condensation method includes preparing an optical fiber including a tip end provided with a photothermal conversion material and arranging the tip end at a position where convection is produced in the liquid as a result of introduction of light into the optical fiber to heat the liquid, the light having a wavelength included in a range of an absorption wavelength of the photothermal conversion material.

(12) The arranging the tip end includes arranging the tip end at a position where light-induced force in addition to the convection is produced in the liquid. The light-induced force includes at least one of light-induced force originating from light that passes through the photothermal conversion material and light-induced force originating from evanescent waves induced by light that propagates through the optical fiber at a surface of a substrate where the liquid is held.

(13) A microscopic object condensation method according to a third aspect of the present disclosure condenses a plurality of microscopic objects dispersed in liquid. The microscopic object condensation method includes preparing an optical fiber including a tip end and arranging the tip end at a position where light-induced force is produced in the liquid in introduction of light into the optical fiber. The light-induced force includes at least one of light-induced force originating from light emitted from the tip end and light-induced force originating from evanescent waves induced by light that propagates through the optical fiber at a surface of a substrate where the liquid is held.

(14) A microscopic object condensation kit according to a fourth aspect of the present disclosure includes a substrate configured to hold on a main surface, liquid where a plurality of microscopic objects are dispersed and an optical fiber including a tip end provided with a photothermal conversion material. The optical fiber is configured such that the tip end is arranged in the liquid when the liquid is held on the main surface.

(15) The photothermal conversion material has a color changed by variation in output of light that propagates through the optical fiber.

(16) The tip end of the optical fiber has a shape of a perfect circle.

(17) The optical fiber is a multi-mode fiber.

(18) A microscopic object condensation system according to a fifth aspect of the present disclosure includes an optical fiber, an adjustment mechanism, and a light source. The optical fiber includes a first end provided with a photothermal conversion material and a second end. The adjustment mechanism adjusts a position or a height of the first end in liquid where a plurality of microscopic objects are dispersed while the liquid is held in a condensation kit. The light source is optically coupled to the second end, and emits light at a wavelength included in a range of an absorption wavelength of the photothermal conversion material.

(19) The light source causes generation of a microbubble at the first end by heating of the liquid around the first end with light. The microscopic object condensation system further includes an imager that takes an image of a region between the first end and the microbubble and a processor that calculates the number of condensed microscopic objects in the region in accordance with an expression (1) below obtained from the image.

[Expression 1]

$$N = \frac{\pi}{6} h \frac{2r_1^2 + r_1 r_2 - h^2 - 3r_3^2 - r_2^2}{V} \times F \cdots (1)$$

In the expression (1), $N$ represents the number of condensed microscopic objects. h represents a height of a condensation region where the plurality of microscopic objects are condensed. $r_1$ represents a distance between a virtual central axis of the microbubble that extends perpendicularly to an end surface of the first end and an outer circumferential portion of the condensation region. $r_2$ represents a distance between the central axis and an inner circumferential portion of the condensation region. $r_3$ represents a distance between the central axis and a portion of the condensation region corresponding to the height. V represents a volume of each of the plurality of microscopic objects. F represents a fill factor of a hexagonal close-packed structure.

(20) The light source causes generation of a microbubble at the first end by heating of the liquid around the first end with light. The microscopic object condensation system further includes an imager that takes an image of a region between the first end and the microbubble and a processor that calculates the number of condensed microscopic objects in the region in accordance with an expression (2) below obtained from the image.
[Expression 2]

$$N = \frac{\pi}{6}\mathrm{h}\frac{-r_1^2+r_1r_2-h^2-3r_3^2+2r_2^2}{V} \times \mathrm{F} \quad \cdots \ (2)$$

In the expression (2), N represents the number of condensed microscopic objects. A represents a height of a condensation region where the plurality of microscopic objects are condensed. $r_1$ represents a distance between a virtual central axis of the microbubble that extends perpendicularly to an end surface of the first end and an outer circumferential portion of the condensation region. $r_2$ represents a distance between the central axis and an inner circumferential portion of the condensation region, $r_3$ represents a distance between the central axis and a portion of the condensation region corresponding to the height. $V$ represents a volume of each of the plurality of microscopic objects. F represents a fill factor of a hexagonal close-packed structure.

ADVANTAGEOUS EFFECTS OF INVENTION

According to the present disclosure, microscopic objects can be condensed at any location in liquid.

BRIEF DESCRIPTION OF DRAWINGS

Fig. 1 is an overall configuration diagram of a microscopic object condensation system according to a first embodiment of the present disclosure.
Fig. 2 is a perspective view schematically showing a configuration of a condensation kit 11 and an optical fiber.
Fig. 3 is a cross-sectional view of the condensation kit and the optical fiber along the line III-III in Fig. 2.
Fig. 4 is a flowchart showing a processing procedure in microscopic object condensation processing in the first embodiment.
Fig. 5 is a diagram for illustrating a method of forming a metallic thin film.
Fig. 6 is a diagram showing an image of an actually prepared optical fiber.
Fig. 7 is a diagram for illustrating a microscopic object condensation mechanism in a condensation step.
Fig. 8 is a diagram showing variation in laser output from a fiber end in each of cases where the metallic thin film is provided/not provided.
Fig. 9 is a diagram showing an exemplary result of microscopic object condensation between the fiber end and a microbubble.
Fig. 10 is a diagram for illustrating influence by a surfactant.
Fig. 11 is a diagram for illustrating an exemplary method of calculating the number of condensed microscopic objects.
Fig. 12 is a perspective view of a condensation kit according to a second embodiment.
Fig. 13 is a cross-sectional view of the condensation kit along the line XIII-XIII in Fig. 12.
Fig. 14 is a schematic diagram for illustrating arrangement of the fiber end.
Fig. 15 is a diagram for illustrating another exemplary method of calculating the number of condensed microscopic objects.
Fig. 16 is a first diagram in which results of condensation of microscopic objects are chronologically aligned in an example where the fiber end is arranged at the center of a sample.
Fig. 17 is a second diagram in which the results of condensation of microscopic objects are chronologically aligned in the example where the fiber end is arranged at the center of the sample.
Fig. 18 is a third diagram in which the results of condensation of microscopic objects are chronologically aligned in the example where the fiber end is arranged at the center of the sample.
Fig. 19 is a diagram showing exemplary results of condensation of microscopic objects in the example where the fiber end is arranged at the center of the sample.
Fig. 20 is a diagram where the results of condensation of microscopic objects are summarized in the example where the fiber end is arranged at the center of the sample.
Fig. 21 is a fluorescent observation image showing the results of condensation of microscopic objects in the example where the fiber end is arranged at the center of the sample.
Fig. 22 is a diagram showing microscopic object condensation efficiency in the example where the fiber end is arranged at the center of the sample.
Fig. 23 is a diagram showing exemplary results of condensation of microscopic objects in an example where the fiber end is arranged at a bottom portion of the sample.

Fig. 24 is a diagram showing a fluorescent observation image of results of condensation of microscopic objects in the example where the fiber end is arranged at the bottom portion of the sample.

Fig. 25 is a diagram showing size dependency of the results of condensation of microscopic objects in the example where the fiber end is arranged at the bottom portion of the sample.

Fig. 26 is a diagram showing concentration dependency of the results of condensation of microscopic objects in the example where the fiber end is arranged at the bottom portion of the sample.

Fig. 27 is a diagram showing a result of analysis of how heat convection flows in the example where the fiber end is arranged at the bottom portion of the sample.

Fig. 28 is a diagram showing a transmittance ratio and an extinction ratio of laser beams when a drive current of a laser light source is varied.

Fig. 29 is a diagram showing an image obtained by imaging of the fiber end before and after variation in drive current of the laser light source.

Fig. 30 is a diagram showing successive images of results of condensation of microscopic objects in an example where a metallic thin film is not formed at the fiber end.

Fig. 31 is a diagram showing successive images of results of condensation of microscopic objects in an example where a metallic thin film is formed at the fiber end.

Fig. 32 is a first diagram where fluorescent observation images of results of condensation of bacteria are chronologically aligned.

Fig. 33 is a second diagram where fluorescent observation images of the results of condensation of bacteria are chronologically aligned.

Fig. 34 is a diagram for comparison of the fluorescent observation images of the results of condensation of bacteria between before and after irradiation with laser beams.

Fig. 35 is a diagram showing results of observation of bacteria after irradiation with laser beams is stopped.

Fig. 36 is a diagram showing a fluorescent observation image of a result of condensation of nanodiamond.

Fig. 37 is a diagram where three manners of optical condensation are organized.

Fig. 38 is a diagram for illustrating a mechanism of long-distance optical condensation.

Fig. 39 is a diagram where fluorescent observation images of results of optical condensation in a comparative example are chronologically aligned.

Fig. 40 is a first diagram where fluorescent observation images of results of optical condensation in the present example are chronologically aligned.

Fig. 41 is a second diagram where fluorescent observation images of the results of optical condensation in the present example are chronologically aligned.

DESCRIPTION OF EMBODIMENTS

<Explanation of Terms>

In the present disclosure and an embodiment, a "nanometer order" includes a range from 1 nm to 1000 nm (= 1 $\mu$m). A "micrometer order" includes a range from 1 $\mu$m to 1000 $\mu$m (= 1 mm). Therefore, a "range from the nanometer order to the micrometer order" includes a range from 1 nm to 1000 $\mu$m. The "range from the nanometer order to the micrometer order" may typically represent a range from several nanometers to several hundred micrometers, preferably a range from 100 nm to 100 $\mu$m, and more preferably a range from several hundred nanometers to several ten micrometers.

In the present disclosure and the embodiment, the term "microscopic object" means an object having a size within the range from the nanometer order to the micrometer order. A shape of the microscopic object is not particularly limited, and it may be, for example, in a spherical shape, a shape of an oval sphere, or a rod shape (a pole shape). When the microscopic object is in the shape of the oval sphere, at least one of a length in a direction of a major axis and a length in a direction of a minor axis of the oval sphere should only be within the range from the nanometer order to the micrometer order. When the microscopic object is in the rod shape, at least one of a width and a length of the rod should only be within the range from the nanometer order to the micrometer order.

Examples of microscopic objects include a metallic nanoparticle, a metallic nanoparticle assembly, a metallic nanoparticle condensed structure body, a semiconductor nanoparticle, an organic nanoparticle, a resin bead, and a particulate matter (PM). The "metallic nanoparticle" refers to a metallic particle having a size of the nanometer order. The "metallic nanoparticle assembly" refers to an assembly formed by aggregation of a plurality of metallic nanoparticles. The "metallic nanoparticle condensed structure body" refers, for example, to a structure body in which a plurality of metallic nanoparticles are fixed to a surface of a substrate (a resin bead etc.) with an interactive site being interposed and arranged at intervals not larger than a diameter of each metallic nanoparticle with gaps being interposed thereamong. The "semiconductor nanoparticle" refers to a semiconductor particle having a size of the nanometer order. The "organic nanoparticle" refers to a particle composed of an organic compound and having a size of the nanometer order. The "resin bead" refers to a particle composed of a resin and having a size within the range from the nanometer order to the micrometer order. The "PM" refers to a particulate substance having a size of the micrometer order. Examples of the PM include PM2.5 and a suspended particulate matter (SPM).

The microscopic object may be a biologically originated substance (a biological substance). More specifically, the microscopic object may include cells, microorganisms (bacteria, fungi, etc.), a drug, a biopolymer (protein, nucleic acid, lipid, polysaccharide, etc.), an antigen (allergen etc.), and a virus.

The term "microbubble" in the present disclosure and the embodiment means an air bubble of the micrometer order.

In the present disclosure and the embodiment, "light-induced force" is used as collective denotation of dissipative force, gradient force, and inter-object light-induced force. Dissipative force refers to force produced by momentum of light being given to a substance in a dissipative process such as light scattering or light absorption. Gradient force refers to force to move a substance to an electromagnetic potential stabilization point when a substance where light-induced polarization has occurred is placed in non-uniform electromagnetic field. Inter-object light-induced force refers to the sum of force originating from vertical electric field and force originating from transverse electric field (radiation field) produced by induced polarization in a plurality of photoexcited substances. Light-induced force may also be read as a light-induced pressure (light-induced force per unit area).

In the present disclosure and the embodiment, a "visible range" means a wavelength range from 360 nm to 760 nm. A "near infrared range" means a wavelength range from 760 nm to 2 $\mu$m.

An embodiment of the present disclosure will be described below in detail with reference to the drawings. The same or corresponding elements in the drawings have the same reference characters allotted and description thereof will not be repeated. In the description below, an x direction and a y direction represent a horizontal direction below. The x direction and the y direction are orthogonal to each other. A z direction represents a vertical direction. An orientation of gravity is downward in the z direction. An upward direction in the z direction may be abbreviated as "upward" and a downward direction in the z direction may be abbreviated as "downward".

[First Embodiment]

<Overall Configuration>

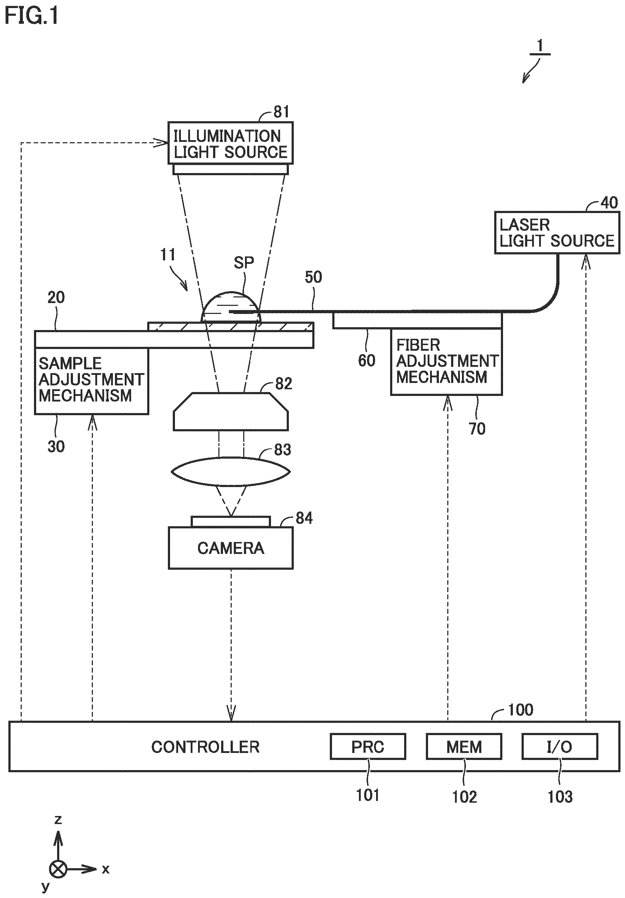

Fig. 1 is an overall configuration diagram of a microscopic object condensation system according to a first embodiment of the present disclosure. A condensation system 1 includes a condensation kit 11, a sample stage 20, a sample adjustment mechanism 30, a laser light source 40, an optical fiber 50, a fiber stage 60, a fiber adjustment mechanism 70, an illumination light source 81, an objective lens 82, a lens 83, a camera 84, and a controller 100.

Condensation kit 11 is configured to hold a sample (shown with SP). The sample is a liquid sample where a plurality of microscopic objects are dispersed.

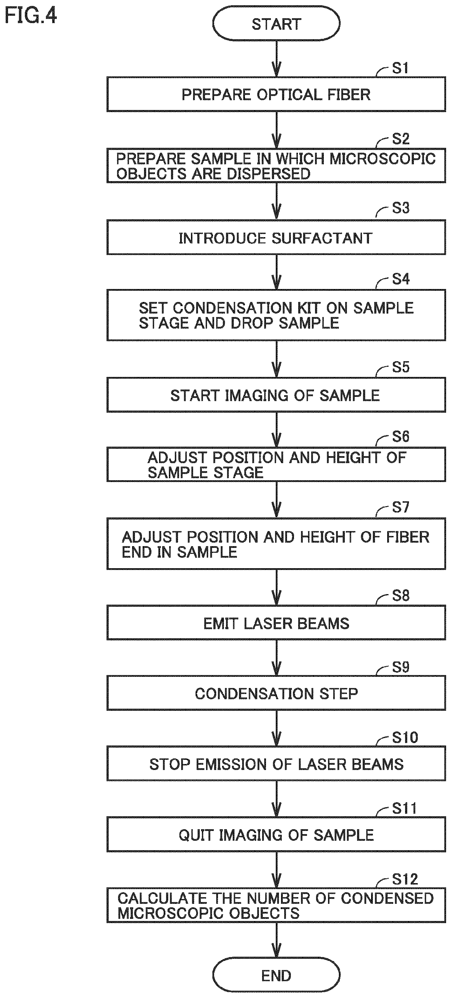

Sample stage 20 is configured such that condensation kit 11 can be placed thereon. Though not shown, a large number of condensation kits 11 may be prepared. The large number of condensation kits 11 are sequentially placed on sample stage 20 and condensation processing (see Fig. 4) which will be described later is performed.

Sample adjustment mechanism 30 is, for example, an XYZ-axis stage. Sample adjustment mechanism 30 adjusts a position in the horizontal direction and a height in the vertical direction of sample stage 20 in accordance with a command from controller 100. Relative positional relation between condensation kit 11 and objective lens 82 or relative positional relation between condensation kit 11 and optical fiber 50 can thus be adjusted.

Laser light source 40 emits laser beams of continuous waves (CW) in accordance with a command from controller 100. A wavelength of laser beams is included in a range of an absorption wavelength of a metallic thin film 52 (which will be described later) formed in condensation kit 11, and it is, for example, a wavelength in a near infrared range.

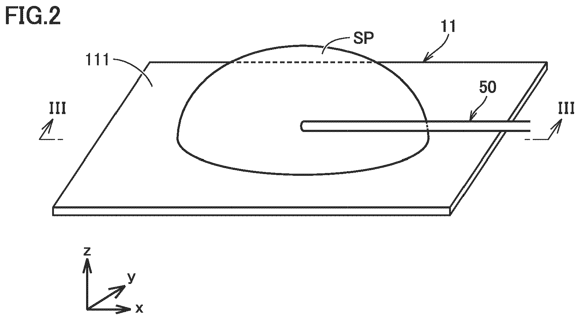

Optical fiber 50 guides laser beams emitted from laser light source 40 to a sample on condensation kit 11. The configuration of condensation kit 11 and optical fiber 50 will be described in detail with reference to Figs. 2 to 5.

Fiber stage 60 is configured such that optical fiber 50 can be placed thereon. Since optical fiber 50 may be damaged or contaminated, it is a consumable article. Therefore, fiber stage 60 is desirably configured such that optical fiber 50 is readily replaceable.

Fiber adjustment mechanism 70 is, for example, an XYZ-axis stage. Fiber adjustment mechanism 70 adjusts a position and a height of optical fiber 50 in accordance with a command from controller 100.

Thus, condensation system 1 shown in Fig. 1 can adjust relative positional relation between condensation kit 11 and optical fiber 50 with any of sample adjustment mechanism 30 and fiber adjustment mechanism 70. Therefore, both of sample adjustment mechanism 30 and fiber adjustment mechanism 70 correspond to the "adjustment mechanism" according to the present disclosure. Condensation system 1, however, may be configured to include only any one of sample adjustment mechanism 30 and fiber adjustment mechanism 70.

Illumination light source 81 emits white light for irradiation of the sample on condensation kit 11. In one example, a halogen lamp can be employed as illumination light source 81. White light emitted from illumination light source 81 passes through the sample. Objective lens 82 takes in white light that passes through the sample. Lens 83 condenses white light taken in by objective lens 82 and guides condensed white light to camera 84.

Camera 84 includes, for example, charge coupled device (CCD) image sensors or complementary metal oxide semiconductor (CMOS) image sensors. Camera 84 takes an image of the sample on condensation kit 11 in accordance with a command from controller 100 and outputs the taken image to controller 100. Images taken by camera 84 may be still images or moving images. Camera 84 corresponds to the "imager" according to the present disclosure.

Controller 100 includes a processor 101 such as a central processing unit (CPU), a memory 102 such as a read only memory (ROM) and a random access memory (RAM), and an input and output port 103 for input and output of various signals. Controller 100 controls each component (sample adjustment mechanism 30, laser light source 40, fiber adjustment mechanism 70, illumination light source 81, and camera 84) in condensation system 1. In addition, controller 100 calculates the number of microscopic objects condensed in the sample based on the image taken by camera 84. This calculation method will be described later.

An optical system (illumination light source 81, objective lens 82, lens 83, and camera 84) for imaging of the sample on condensation kit 11 is merely by way of example. The optical system of condensation system 1 may be configured, for example, such that the sample is irradiated with white light from illumination light source 81 from below and camera 84 takes an image of the sample from above. The optical system of condensation system 1 may include another optical component (a mirror, a dichroic mirror, a beam splitter, a filter, an optical fiber, or the like) instead of or in addition to objective lens 82 and lens 83.

<Condensation Kit and Optical Fiber>

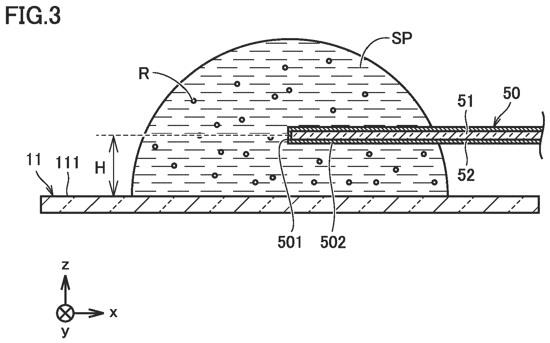

Fig. 2 is a perspective view schematically showing a configuration of condensation kit 11 and optical fiber 50. Fig. 3 is a cross-sectional view of condensation kit 11 and optical fiber 50 along the line III-III in Fig. 2. Figs. 2 and 3 do not show sample stage 20 and fiber stage 60.

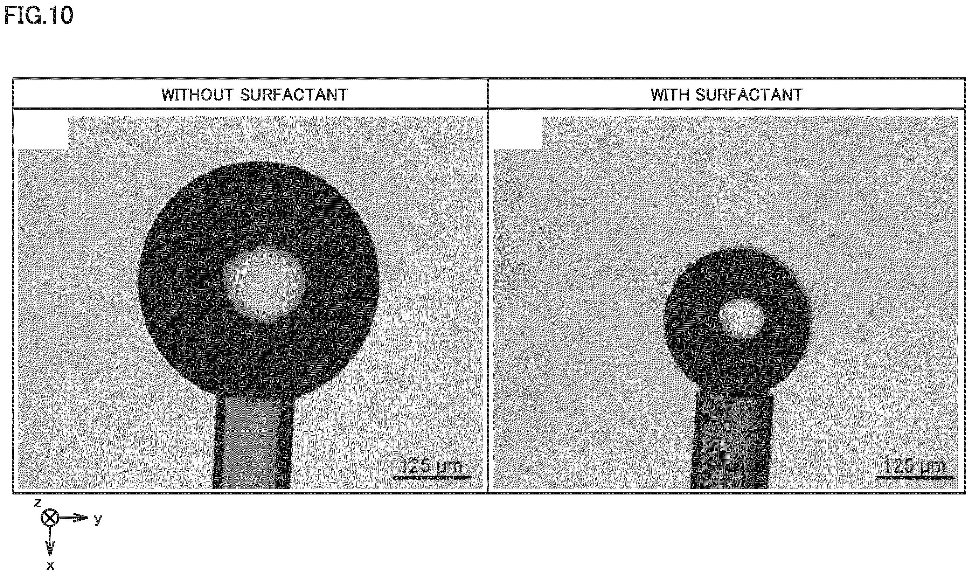

The sample is, for example, a liquid sample in which resin beads (shown with R) are dispersed. In an example which will be described later, polystyrene particles are employed as resin beads. Though a type of liquid (dispersion medium) is not particularly limited, water is adopted in this example. A non-ionic surfactant for expediting (see Fig. 10) condensation of resin beads is added to the sample.

Condensation kit 11 is a substrate in a form of a flat plate in the first embodiment. Condensation kit 11 holds the sample on an upper surface (main surface) 111 thereof. A material for condensation kit 11 is transparent to white light. Examples of such a material include quartz and silicone. In this example, a glass substrate (cover glass) is employed as condensation kit 11.

Condensation kit 11 may be a three-dimensional container in which an internal space for holding the sample is provided. Specifically, a columnar glass bottom dish may be employed as condensation kit 11.

Optical fiber 50 has one tip end (a first end) 501 arranged in the sample. Optical fiber 50 has the other tip end (a second end) optically coupled to laser light source 40 (see Fig. 1). Optical fiber 50 is preferably a multi-mode fiber, and it allows multi-mode propagation of laser beams incident on the second end to guide them to the first end. Optical fiber 50 includes a propagation path 51 and metallic thin film 52.

Though "optical fiber 50 having the tip end arranged in the sample" encompasses a state where the entire tip end of optical fiber 50 is enclosed in the sample as shown in Figs. 2 and 3, it is not limited as such. A state where the tip end of optical fiber 50 is located at a gas-liquid interface (an interface between the sample and gas therearound) is also encompassed in "optical fiber 50 having the tip end arranged in the sample."

Propagation path 51 includes a core and a clad (neither of which is shown). Propagation path 51 may be composed of quartz glass or plastic.

Metallic thin film 52 is formed to cover propagation path 51. Though Fig. 3 shows an example where metallic thin film 52 is formed at both of tip end (first end) 501 and a side surface of optical fiber 50, metallic thin film 52 should only be formed at least at the tip end of optical fiber 50. Metallic thin film 52 absorbs laser beams from laser light source 40 and converts light energy into thermal energy. More specifically, free electrons at a surface of metallic thin film 52 form surface plasmons and are oscillated by laser beams. Polarization thus occurs. Energy of polarization is converted to energy of lattice vibration as a result of Coulomb interaction between free electrons and nuclei. Consequently, metallic thin film 52 generates heat. This effect is also referred to as a "photothermal effect" below.

A material for metallic thin film 52 is preferably great in photothermal effect (in other words, high in photothermal conversion efficiency) in a wavelength range of laser beams. In the present embodiment, a gold thin film is formed as metallic thin film 52. The material for metallic thin film 52, however, is not limited to gold, and a metal element (for example, silver) other than gold or a metallic nanoparticle assembly structure body (for example, a structure body which is assembly of gold nanoparticles or silver nanoparticles) that may achieve the photothermal effect may be applicable.

A thickness of metallic thin film 52 is determined in terms of design or experimentally, taking into account a wavelength of laser beams, power (laser output) of laser beams, characteristics (a range of an absorption wavelength and photothermal conversion efficiency) of a material for metallic thin film 52, and the like. When the wavelength of laser beams is within the near infrared range and laser output is several hundred milliwatts, the thickness of metallic thin film 52 may be set to the order of nanometers. In an example which will be described later, when a central wavelength of laser beams is 980 nm and laser output is from 200 mW to 500 mW, the thickness of metallic thin film 52 is set to 10 nm.

Tip end (first end) 501 of optical fiber 50 is also denoted as a "fiber end 501" below. A height of fiber end 501 with upper surface 111 of condensation kit 11 being defined as the reference is denoted as "H". As controller 100 controls sample adjustment mechanism 30 and/or fiber adjustment mechanism 70, height H can be set to any value.

A position of fiber end 501 may be fixed such that fiber end 501 is located in the sample when the sample is held on/over upper surface 111. For example, condensation kit 11 may include a holder (not shown) for optical fiber 50. The holder is arranged on upper surface 111 to fix the position and the height of fiber end 501 to values set in advance.

In Figs. 2 and 3, optical fiber 50 is inserted into the sample in the horizontal direction. This is because the optical system for imaging of the sample is constructed in the vertical direction (see Fig. 1), and hence insertion of optical fiber 50 in the horizontal direction facilitates imaging of fiber end 501. The direction of insertion of optical fiber 50 is not limited to the horizontal direction. Optical fiber 50 may be inserted into the sample from any direction such as the vertical direction or a diagonal direction.

<Flowchart>

Fig. 4 is a flowchart showing a processing procedure in microscopic object condensation processing in the first embodiment. A series of processing shown in this flowchart is performed as being called from a not-shown main routine when a predetermined condition is satisfied (for example, when condensation system 1 accepts a start operation from a measurer). Though each step is basically performed by software processing by controller 100, it may be performed by hardware (electric circuitry) arranged in controller 100. The step will be abbreviated as S below.

In S1, optical fiber 50 is prepared. For example, optical fiber 50 can be prepared, for example, by pre-treatment by the measurer for forming metallic thin film 52 in an optical fiber yet to be treated. Dedicated optical fiber 50 provided with metallic thin film 52 at the tip end may be available in the market.

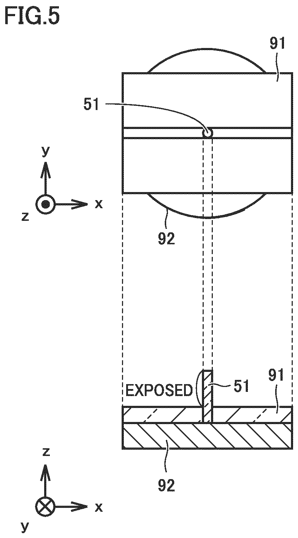

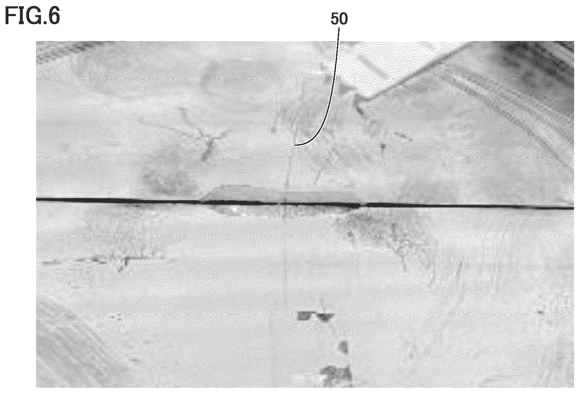

Fig. 5 is a diagram for illustrating a method of forming metallic thin film 52. Fig. 5 shows a top view (an upper figure) and a cross-sectional view (a lower figure) showing a state in formation of metallic thin film 52. Initially, a coating at the tip end (first end) of propagation path 51 is removed. Propagation path 51 is then fixed on slide glass 91 such that a portion from which the coating was removed is slightly (for example, by 1 to 2 cm) exposed. Metallic thin film 52 (the gold thin film in this example) is then formed in the exposed portion by ion sputtering. Metallic thin film 52 can be formed also by using another known method such as electroless plating. Thereafter, another tip end (second end) is optically coupled to an optical connector (for example, an FC/PC connector). Fig. 6 is a diagram showing an image of actually prepared optical fiber 50.

Instead of metallic thin film 52, a material other than metal high in light absorption factor at the wavelength of laser beams may be arranged at the tip end of optical fiber 50. Examples of such a material include a material close to a black body (for example, a carbon nanotube black body). A region where metallic thin film 52 is formed or a region where a carbon nanotube black body or the like is arranged corresponds to the "photothermal conversion region" according to the present disclosure.

Referring back to Fig. 4, in S2, a sample where microscopic objects are dispersed is prepared. Though details will be described later, a surfactant is desirably introduced into the sample (S3). The sample prepared in S2 and S3 is accommodated in a not-shown sample supply portion (for example, a dispenser). Processing in S2 and S3 is performed by the measurer.

In S4, controller 100 has condensation kit 11 placed on sample stage 20. This processing may be performed, for example, by a feed mechanism (not shown) to feed condensation kit 11. Furthermore, controller 100 controls a sample supply portion (not shown) to drop the sample such that an appropriate amount of sample is held on upper surface 111 of condensation kit 11 as shown in Figs. 2 and 3. An amount of drop of the sample may be, for example, a small amount approximately from several ten microliters to several hundred microliters, or more than that.

In S5, controller 100 starts imaging of the sample. Specifically, controller 100 controls illumination light source 81 to emit white light to irradiate the sample on condensation kit 11 therewith and controls camera 84 to start imaging of the sample.

In S6, controller 100 controls sample adjustment mechanism 30 to adjust the position and the height of sample stage 20 to a position and a height suitable for imaging by camera 84. This processing may be performed by processing by controller 100, of the image taken by camera 84. Controller 100 can adjust the position of sample stage 20, for example, such that the sample is located around the center of the image and can adjust the height of sample 20 such that the sample is in focus. This processing may be performed by a manual operation by the measurer.

In S7, controller 100 controls fiber adjustment mechanism 70 to adjust the position and the height of fiber end 501 in the sample. The position of fiber end 501 may be adjusted, for example, by extraction of fiber end 501 from the image taken by camera 84 according to an image processing technique for pattern recognition. An initial value H0 of height H of fiber end 501 is set at the time of placement of optical fiber 50 on fiber stage 60, and it has already been known. Therefore, the height of fiber end 501 may be adjusted by addition of an amount of change $\Delta H$ in a direction of height by fiber adjustment mechanism 70 to initial value H0. Amount of change $\Delta H$ can also take a negative value, without being limited to a positive value. Controller 100 may control sample adjustment mechanism 30 instead of fiber adjustment mechanism 70 to adjust the position and the height of fiber end 501.

In S8, controller 100 controls laser light source 40 to start irradiation of the sample with laser beams.

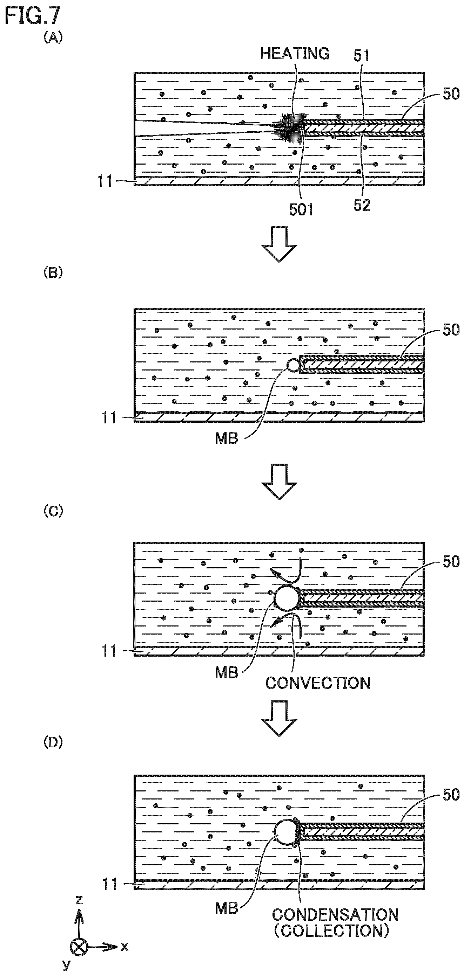

In S9, microscopic objects are condensed in the vicinity of fiber end 501 through a condensation step. The condensation step will be described in detail with reference to Figs. 7 to 10.

In S10, controller 100 controls laser light source 40 to stop irradiation of the sample with laser beams.

In S11, controller 100 quits imaging of the sample. Specifically, controller 100 controls illumination light source 81 to stop emission of white light and controls camera 84 to stop imaging of the sample.

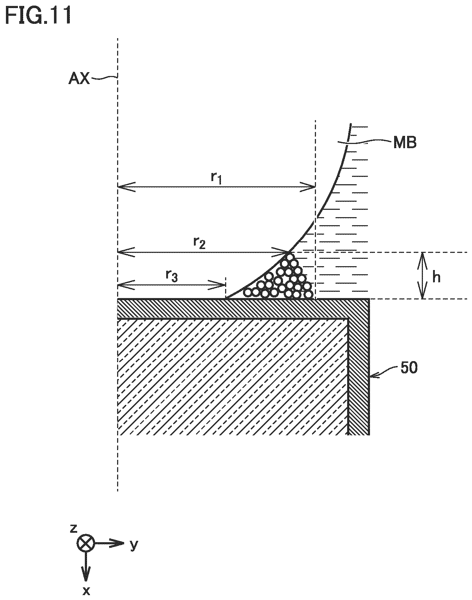

In S12, controller 100 calculates the number of condensed microscopic objects in the vicinity of fiber end 501 based on the image taken by camera 84. This calculation method will be described with reference to Fig. 11. The series of processing thus ends.

Processing in S5, S6, and S11 is processing for imaging of a manner of condensation of microscopic objects, and it is not essential for condensation of microscopic objects. Microscopic objects can be condensed also when a flowchart not including the processing in S5, S6, and S11 is performed.

<Mechanism>

Fig. 7 is a diagram for illustrating a microscopic object condensation mechanism in the condensation step (processing in S9 in Fig. 4). Metallic thin film 52 has been formed at fiber end 501. Therefore, when irradiation with laser beams is started, a portion around (in the vicinity of) fiber end 501 is locally heated owing to the photothermal effect of metallic thin film 52. Then, the dispersion medium (water in this example) around fiber end 501 boils or the like, and a microbubble (shown with MB) is generated at fiber end 501. Microbubble grows with lapse of time.

With irradiation with laser beams, regular heat convection is steadily produced in the dispersion medium in addition to microbubble. Heat convection may include buoyant convection and Marangoni convection. Reference to PTLs 1 and 2 may be made for detailed reasons for production of heat convection.

A direction of heat convection is a direction once heading toward fiber end 501 and thereafter deviating from fiber end 501 as shown with an arrow in the figure. Microscopic objects are carried over heat convection toward fiber end 501 and caught by microbubble. More specifically, a "stagnation region" which is a region where a flow velocity of heat convection is substantially zero is produced between microbubble and fiber end 501. As microscopic objects carried over heat convection are caught in the stagnation region, microscopic objects are condensed in the vicinity of fiber end 501. Microbubble functions as a stopper to hold back microscopic objects, and becomes a collection site where microscopic objects are collected.

A function to condense microscopic objects dispersed in the sample in the vicinity of fiber end 501 according to the above mechanism can also be called "optical condensation." "Condensation" of microscopic objects means that a concentration of microscopic objects around fiber end 501 becomes higher than a concentration of microscopic objects in another region in the sample. Collection of microscopic objects in the stagnation region can also be defined as condensation of microscopic objects. As will be described later in a second embodiment, production of microbubble is not essential for optical condensation. Without production of microbubble, heat convection can condense microscopic objects in the vicinity of fiber end 501.

A function for collection of microscopic objects by optical condensation at a collection site such as a solid-liquid interface between fiber end 501 and a dispersion medium, a gas-liquid interface between microbubble and a dispersion medium, a three-phase boundary of solid-liquid-gas may also be called "optical collection."

Controller 100 can control at least one of sample adjustment mechanism 30 and fiber adjustment mechanism 70 to move fiber end 501 to any position and height in the sample. Therefore, according to the present embodiment, microscopic objects can be condensed at any location in the sample.

[First Example]

<Extinction of Laser Beams>

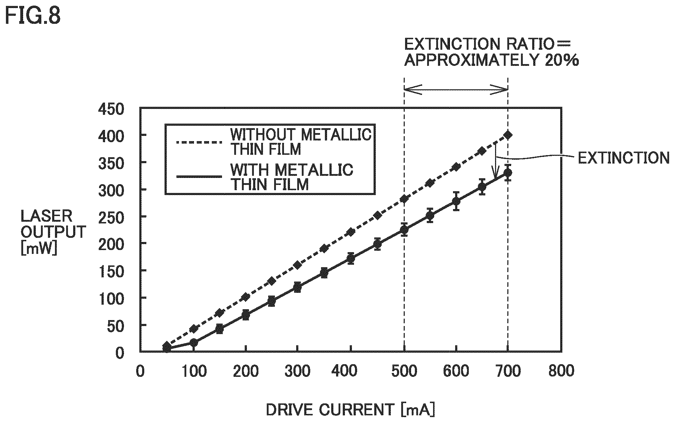

Fig. 8 is a diagram showing variation in laser output from fiber end 501 in each of cases where metallic thin film 52 is provided/not provided. The abscissa represents a drive current supplied to laser light source 40. The ordinate represents laser output (power of laser beams that are introduced from laser light source 40 into optical fiber 50, propagate through optical fiber 50, and are outputted from fiber end 501).

Fig. 8 shows for the purpose of comparison, a result of measurement (see a dashed line) in a configuration where metallic thin film 52 is not formed at fiber end 501, in addition to a result of measurement (see a solid line) in a configuration where metallic thin film 52 is formed at fiber end 501. A difference in laser output between these two results of measurement corresponds to an amount of extinction (= an amount of absorption + an amount of reflection) of laser beams attributed to metallic thin film 52. This difference can be concluded as evidence of formation (successful film formation) of metallic thin film 52 at fiber end 501.

In a current range from 500 mA to 700 mA, the extinction ratio of laser beams was as high as approximately 20%. The extinction ratio refers to a ratio of an amount of extinction of laser beams to laser output in the configuration where metallic thin film 52 is not formed at fiber end 501. The photothermal effect of metallic thin film 52 is dependent on an amount of absorption of laser beams. Therefore, as the extinction ratio of laser beams is higher, microbubble may highly efficiently be generated. It was actually confirmed that microbubble was readily generated and grown in this current range.

[Second Example]

<Condensation of Polystyrene Particles>

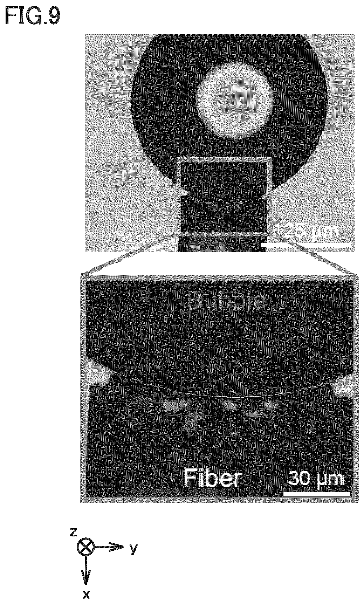

Fig. 9 is a diagram showing an exemplary result of condensation of microscopic objects between fiber end 501 and microbubble. Polystyrene particles each having a diameter of 1 $\mu$m were employed as microscopic objects. A concentration of the polystyrene particles in the sample was $4.5 \times 510^7$ [particles/mL]. A volume of the sample was set to 20 $\mu$L. The drive current of laser light source 40 was set to 700 mA. A time period for irradiation with laser beams was set to 60 seconds. Optical fiber 50 had a diameter of 125 $\mu$m. In this measurement and each measurement which will be described later, the drive current of 700 mA corresponds to laser output of 401 W, the drive current of 600 mA corresponds to laser output of 344 mW, and the drive current of 500 mA corresponds to laser output of 287 mW.

Microbubble larger than the diameter of fiber end 501 was generated at fiber end 501. It could also be confirmed that polystyrene particles were condensed (collected) between fiber end 501 and microbubble.

As shown in Fig. 9, fiber end 501 is desirably formed perpendicularly to the side surface of optical fiber 50. In other words, fiber end 501 desirably has a shape (a cross-sectional shape perpendicular to the side surface) in a perfect circle rather than an oval. Microbubble can thus be held at fiber end 501 in a stable manner.

[Third Example]

<Influence by Surfactant>

Fig. 10 is a diagram for illustrating influence by a surfactant. Conditions for measurement are the same as those described with reference to Fig. 9. Tween®20 was employed as the surfactant. A concentration of the surfactant was set to $9.05 \times 10^{-5}$ M, which is close to the critical micelle concentration of Tween®20.

In an example where the surfactant was not contained in the sample (left figure), the number of condensed polystyrene particles was smaller than in an example where the surfactant was contained in the sample (right figure). This may be because, when the surfactant was not contained, microbubble was too large and the flow velocity of heat convection was too high. Too large a microbubble leads to decrease in size of the stagnation region provided between fiber end 501 and microbubble which contributes to catching of microscopic objects. Too high a flow velocity of heat convection tends to lead to difficulty in catching polystyrene particles in the stagnation region. Consequently, the number of condensed polystyrene particles may become small.

The surfactant adsorbs to the surface of microbubble to suppress growth of microbubble and reduces surface tension of microbubble to suppress the flow velocity of Marangoni convection. Therefore, by introduction of the surfactant into the sample, excessive growth of microbubble can be suppressed and the flow velocity of heat convection can moderately be suppressed, so that condensation of polystyrene particles in the stagnation region can be promoted. In particular, by preparing the concentration of the surfactant to the critical micelle concentration (or a concentration close thereto), condensation of polystyrene particles can further be promoted.

[Fourth Example]

<Calculation of the Number of Condensed Particles>

Fig. 11 is a diagram for illustrating an exemplary method of calculating the number of condensed microscopic objects. In this method, the shape of each microscopic object is assumed as being spherical. Furthermore, in a region having a cross-sectional shape of a frustum where microscopic objects are condensed, microscopic objects are assumed to have a hexagonal close-packed structure. According to such a geometrical model, the number N of condensed microscopic objects is calculated in accordance with an expression (1) below.
[Expression 3]

$$N \ = \ \frac{\pi}{6}\mathrm{h}\frac{2r_1^2+r_1r_2-h^2-3r_3^2-r_2^2}{V}\times \mathrm{F} \ \cdots \ (1)$$

In the expression (1), h represents a height of a condensation region where microscopic objects are condensed. $r_1$ represents a distance between a central axis (shown with AX) of microbubble and an outer circumferential portion of the condensation region. The central axis of microbubble refers to a virtual axis that extends perpendicularly to the end surface of optical fiber 50. $r_2$ represents a distance between the central axis of microbubble and a highest portion of the condensation region. $r_3$ represents a distance between the central axis of microbubble and an inner circumferential portion of the condensation region. V represents a volume of each microscopic object. F represents a fill factor of the hexagonal close-packed structure, and it is set approximately to 0.74.

The number N of condensed polystyrene particles in exemplary measurement shown in Figs. 8 to 10 is calculated in accordance with the expression (1) as N = 24785±3212 [particles]. Efficiency in condensation of polystyrene particles is thus calculated as 2.7%±0.4%. Condensation efficiency refers to a ratio of the number N of condensed polystyrene particles to the total number of polystyrene particles in the sample. Condensation efficiency of 2.7% is higher by an order of magnitude than condensation efficiency of 0.15% described in PTL 1. Therefore, according to the first embodiment, it can be concluded that polystyrene particles could highly efficiently be condensed.

As set forth above, in the first embodiment, microscopic objects are condensed in the vicinity of the tip end of optical fiber 50 by making use of the photothermal effect of metallic thin film 52 formed at the tip end of optical fiber 50. The tip end of optical fiber 50 can be adjusted to any position and/or height in the sample. Therefore, according to the first embodiment, microscopic objects can be condensed at any location in the sample. In addition, efficiency in condensation of microscopic objects significantly higher than that in the configurations in PTLs 1 and 2 can be achieved.

[Second Embodiment]

The example in which condensation kit 11 is in the form of the flat plate is described in the first embodiment (see Figs. 2 and 3). Condensation kit 11 can be concluded as being in a structure of an "open system" in that the sample is open to the atmosphere. In the second embodiment, an example in which the condensation kit is in a structure of a "closed system" will be described.

An overall configuration of a microscopic object condensation system according to the second embodiment is equivalent to the overall configuration (see Fig. 1) of microscopic object condensation system 1 according to the first embodiment. A processing procedure in microscopic object condensation processing in the second embodiment is basically equivalent to the processing procedure (see Fig. 4) in the first embodiment. Therefore, detailed description thereof will not be repeated.

<Condensation Kit>

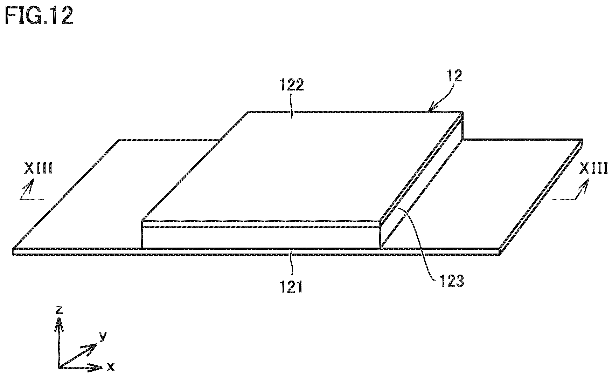

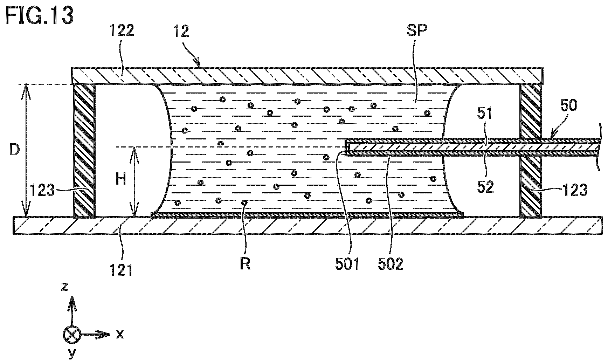

Fig. 12 is a perspective view of the condensation kit according to the second embodiment. Fig. 13 is a cross-sectional view of the condensation kit along the line XIII-XIII in Fig. 12. Referring to Figs. 12 and 13, a condensation kit 12 is configured not only to hold the sample but also to sandwich the sample from above and below in the vertical direction. Condensation kit 12 includes a substrate 121, a cover 122, and a spacer 123.

Substrate 121 is arranged below the sample to hold the sample. A material for substrate 121 is a material (glass, quartz, silicone, or the like) transparent to white light. In this example, a glass substrate (cover glass) is employed as substrate 121.

Cover 122 covers the sample held on substrate 121 from above. Cover 122 is composed of a material transparent to white light, similarly to substrate 121. In this example, a glass substrate is employed also for cover 122.

Spacer 123 is arranged between substrate 121 and cover 122. Spacer 123 fixes cover 122 to substrate 121 and maintains a distance between substrate 121 and cover 122 to a setting value. In this example, a double-faced tape is employed as spacer 123. A material for spacer 123, however, is not particularly limited, and other materials such as resin, rubber, glass, quartz, or silicone may be applicable.

Spacer 123 can function also as a holder that fixes the position and the height of fiber end 501 to values set in advance. For example, a minute groove or through hole through which optical fiber 50 passes can be provided in spacer 123. Condensation kit 12 can thus be configured such that fiber end 501 is located in the sample when the sample is held on/over substrate 121.

The distance between substrate 121 and cover 122 (that is, a sample height) is denoted as "D" below. Height H of fiber end 501 can be adjusted to any value within a range from 0 to D ($0 \leq H \leq D$).

[Fifth Example]

<Arrangement of Fiber End>

A result of assessment of influence by arrangement (specifically, height H) of fiber end 501 on condensation of microscopic objects will be described.

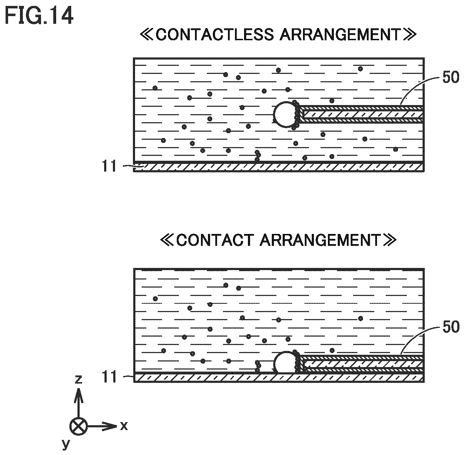

Fig. 14 is a schematic diagram for illustrating arrangement of fiber end 501. As shown in Fig. 14, results of condensation of microscopic objects were compared between an example where fiber end 501 was arranged at a center of the sample (H = D/2) and an example where fiber end 501 was arranged at a bottom portion of the sample (H = 0). Sample height D was set to D = 860 $\mu$m.

When fiber end 501 was arranged at the center of the sample, the propagation path of optical fiber 50 does not come in contact with substrate 11, and hence this arrangement is also denoted as "contactless arrangement." In contrast, when fiber end 501 is arranged at the bottom portion of the sample, the propagation path of optical fiber 50 comes in contact with substrate 11, and hence this arrangement is also denoted as "contact arrangement."

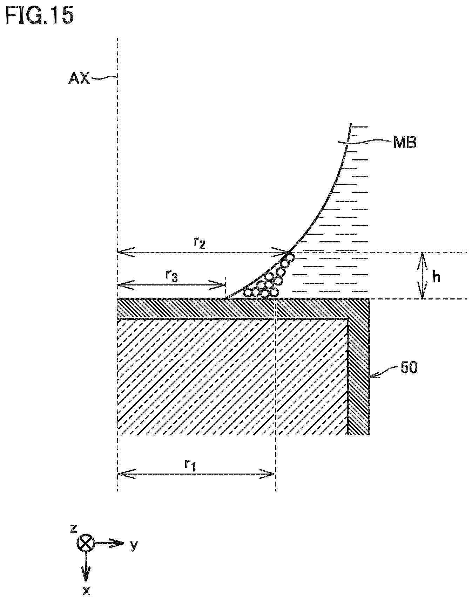

Fig. 15 is a diagram for illustrating another exemplary method of calculating the number of condensed microscopic objects. In exemplary measurement below, since the shape of the condensation region where microscopic objects are condensed was different from the shape shown in Fig. 11, the geometrical model for calculation of the number N of condensed microscopic objects was also modified. Specifically, the number N of condensed microscopic objects was calculated in accordance with an expression (2) below.

[Expression 4]

$$ N = \frac{\pi}{6}\mathrm{h}\frac{-r_1^2 + r_1 r_2 - h^2 - 3r_3^2 + 2r_2^2}{V} \times \mathrm{F} \quad \cdots (2) $$

«Contactless Arrangement»

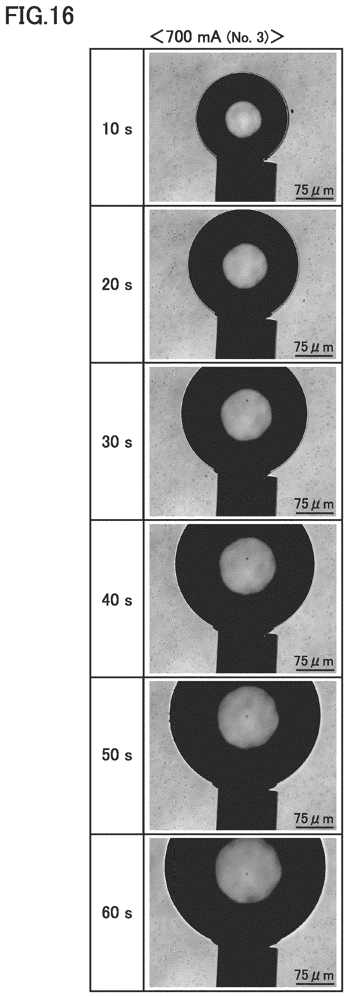

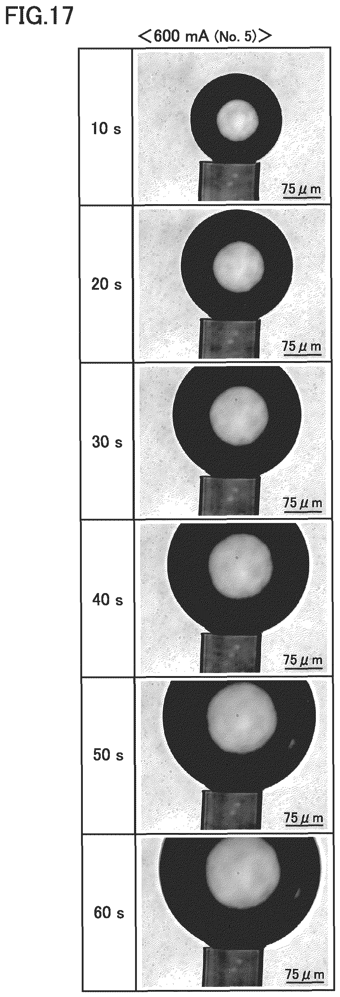

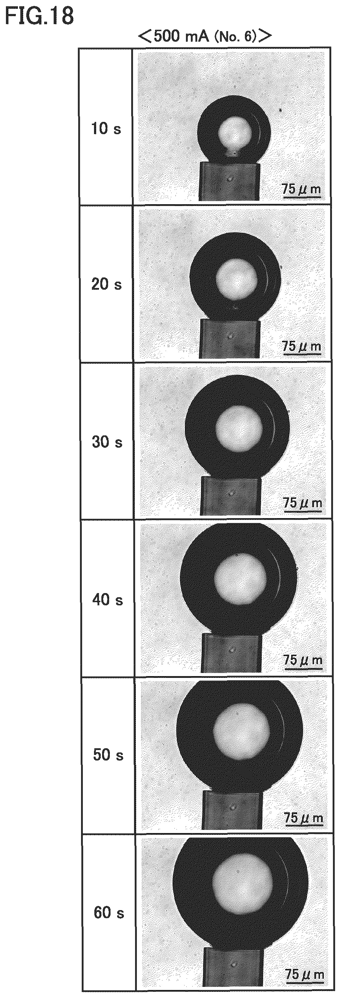

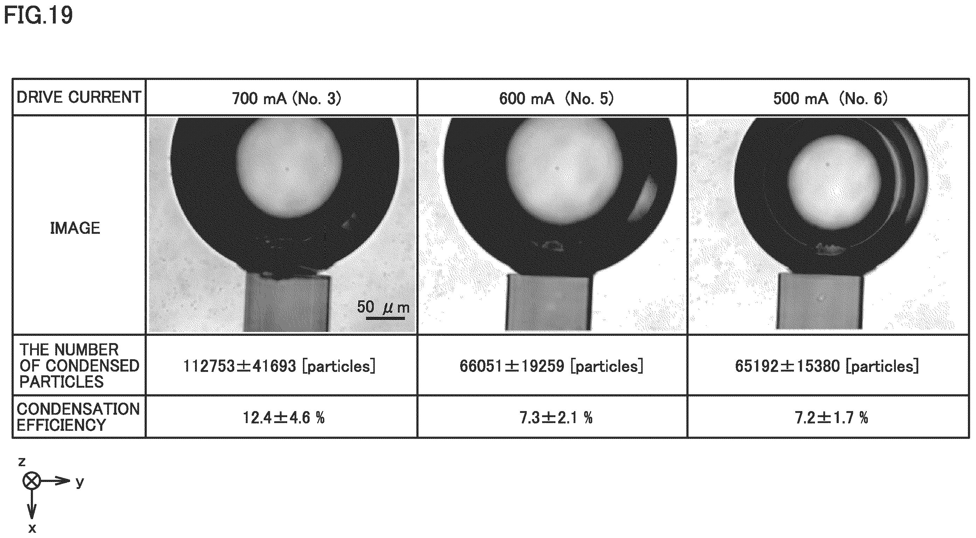

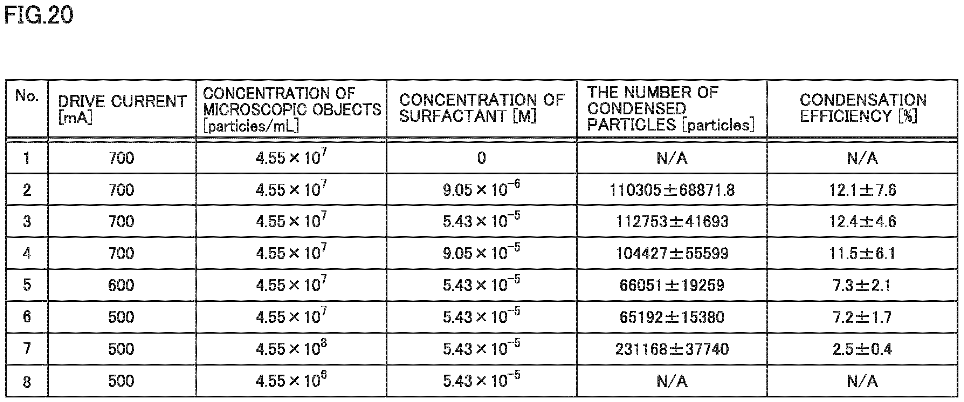

Figs. 16 to 18 are each a diagram in which results of condensation of microscopic objects are chronologically aligned in the example where fiber end 501 is arranged at the center of the sample (contactless arrangement). Fig. 19 is a diagram for comparison of results of condensation of microscopic objects in the example where fiber end 501 is arranged at the center of the sample. Fig. 19 shows an image taken sixty seconds after start of irradiation with laser beams. Fig. 20 is a diagram where the results of condensation of microscopic objects are summarized in the example where fiber end 501 is arranged at the center of the sample.

The drive current of laser light source 40 was set to 700 mA (laser output of 401 mW), 600 mA (laser output of 344 mW), or 500 mA (laser output of 287 mW). Polystyrene particles each having a diameter of 1 $\mu$m were employed. The concentration of polystyrene particles was set within a range from $4.55 \times 10^6$ to $4.55 \times 10^8$ [particles/mL]. The volume of the sample was set to 20 $\mu$L. Tween®20 was employed as the surfactant. The concentration of the surfactant was prepared within a range from 0 to $9.05 \times 10^{-5}$M.

Referring to Figs. 16 to 20, efficiency in condensation of polystyrene particles when the drive current was set to 500 mA (sample numbers 6 to 8) or 600 mA (sample number 5) was lower than that when the drive current was set to 700 mA (sample numbers 2 to 4). When the surfactant was not introduced (sample number 1), polystyrene particles were not condensed even when the drive current was set to 700 mA.

Among sample numbers 2 to 4, the drive current of 700 mA was in common and the concentration of polystyrene particles of $4.55 \times 10^7$ [particles/mL] was also in common, whereas the concentration of the surfactant was different. The condensation efficiency in each of sample numbers 2 to 4 was higher by two orders of magnitude than the condensation efficiency (0.15%) described in PTL 1. Among others, the condensation efficiency of sample number 3 which was intermediate in concentration of the surfactant was highest and also least in variation. Sample number 2 lowest in concentration of the surfactant and sample number 4 highest in concentration of the surfactant were relatively great in variation in condensation efficiency, although they were sufficiently high in condensation efficiency.

As the drive current is greater, the flow velocity of heat convection (buoyant convection and Marangoni convection) produced in the sample is higher. As the concentration of the surfactant is higher, on the other hand, growth of microbubble is suppressed and the flow velocity of heat convection (in particular, Marangoni convection) is suppressed. By optimizing combination between magnitude of the drive current and the concentration of the surfactant, the size of microbubble can be adjusted and the flow velocity of heat convection can be adjusted to the flow velocity suitable for condensation of microscopic objects, so that high condensation efficiency less in variation can be achieved.

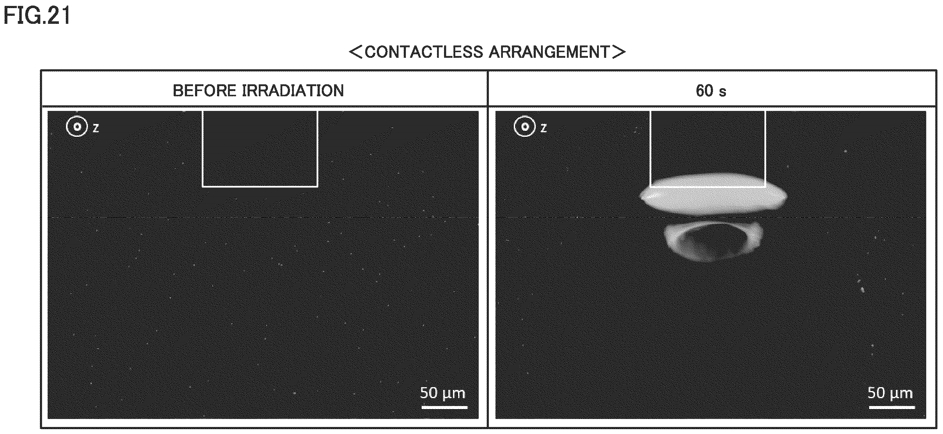

Fig. 21 is a fluorescent observation image showing results of condensation of microscopic objects in the example where fiber end 501 is arranged at the center of the sample (contactless arrangement). The concentration of polystyrene particles each having a diameter of 1 $\mu$m was $4.55\times10^7$ [particles/mL]. The concentration of Tween®20 was set to $5.43\times10^{-5}$ M. Laser output was set to 390 mW. The time period for irradiation with light was set to sixty seconds. In the contactless arrangement, it can be seen that polystyrene particles are condensed at fiber end 501 (more specifically, the three-phase boundary among fiber end 501 (solid phase), the sample (liquid phase), and microbubble (vapor phase)).

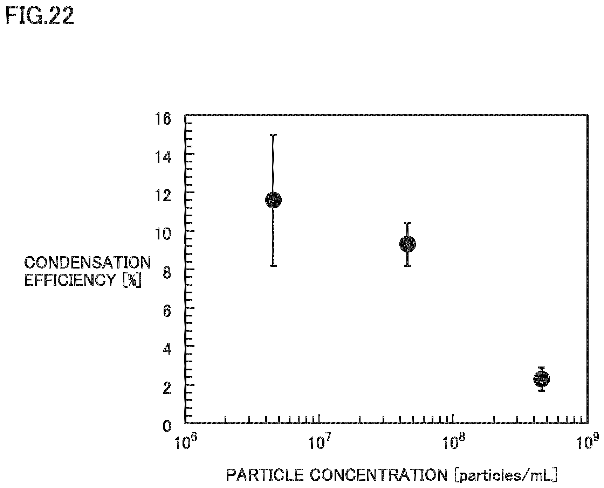

Fig. 22 is a diagram showing efficiency in condensation of microscopic objects in the example where fiber end 501 is arranged at the center of the sample (contactless arrangement). Measurement conditions were in common to those in Fig. 21 except that the concentration of polystyrene particles was set to various values. The abscissa represents the concentration of polystyrene particles. The ordinate represents efficiency in condensation of polystyrene particles. As the concentration of polystyrene particles was lower, condensation efficiency was higher. At the lowest concentration, condensation efficiency of 11.6% was achieved.

<<Contact Arrangement>>

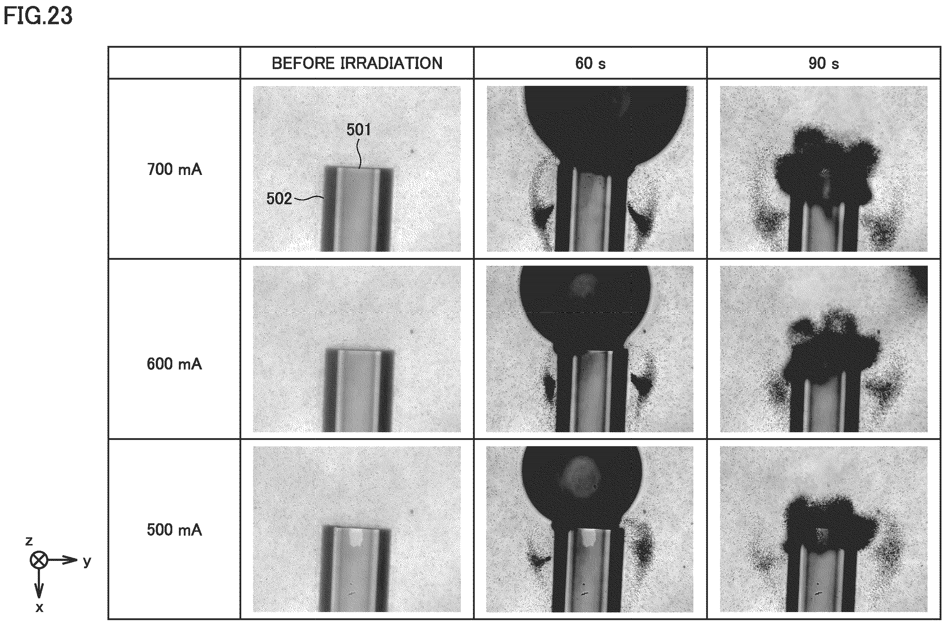

Fig. 23 is a diagram showing exemplary results of condensation of microscopic objects in an example where fiber end 501 is arranged at the bottom portion of the sample (contact arrangement). The drive current of laser light source 40 was set to 700 mA, 600 mA, or 500 mA. Polystyrene particles each having a diameter of 1 $\mu$m were employed. The concentration of polystyrene particles was prepared to $4.55\times10^8$ [particles/mL]. The volume of the sample was set to 20 $\mu$L. Tween®20 was employed as the surfactant. The concentration of the surfactant was prepared to $5.43\times10^{-5}$ M.

In contact arrangement, regardless of whether the drive current was set to any of the three values above, polystyrene particles were condensed on the side surface of optical fiber 50 (which will be denoted as a "fiber side surface 502" below) rather than a portion between fiber end 501 and microbubble. It is estimated that, in contact arrangement, heat convection different from that in contactless arrangement is produced due to substrate 121 located around fiber end 501.

A temperature of the sample on fiber side surface 502 is lower than a temperature of the sample at fiber end 501. Therefore, the contact arrangement is expected to be suitable for condensation of heat-sensitive microscopic objects (a drug or a biological substance such as a biopolymer). For example, by arrangement of cells in the vicinity of fiber side surface 502 and optical condensation of a drug on fiber side surface 502, the optically condensed drug can be introduced into the cells.

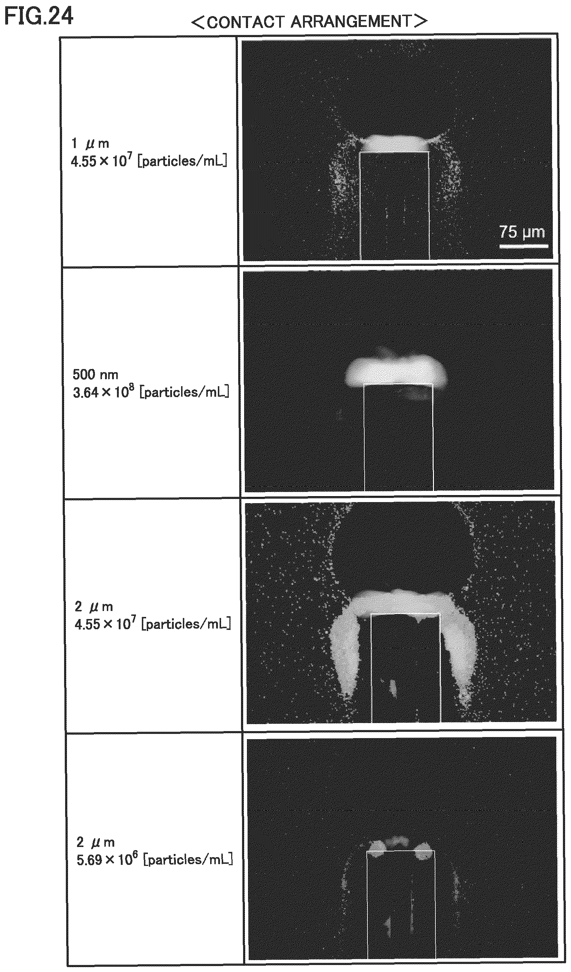

Fig. 24 is a diagram showing a fluorescent observation image of results of condensation of microscopic objects in the example where fiber end 501 is arranged at the bottom portion of the sample (contact arrangement). Four samples different from one another in size and concentration of polystyrene particles were prepared. The concentration of polystyrene particles (NYO) each having a diameter of 500 nm was $3.64\times10^8$ [particles/mL]. The concentration of polystyrene particles (YG) each having a diameter of 1 $\mu$m was $4.5\times510^7$ [particles/mL]. The concentration of polystyrene particles (YG) each having a diameter of 2 $\mu$m was $4.5\times510^7$ [particles/mL] or $5.69\times10^6$ [particles/mL]. The concentration of Tween®20 was $5.43\times10^{-5}$ M. Laser output was set to 320 mW. The time period for irradiation with light was set to sixty seconds.

It can be seen in Fig. 24 that condensation large in scale not only at fiber end 501 but also on fiber side surface 502 is achieved in contact arrangement. It can be concluded that in contactless arrangement, polystyrene particles are condensed at optical fiber 50 (fiber end 501) (see Fig. 21), whereas in contact arrangement, polystyrene particles are condensed at a position relatively distant from optical fiber 50. The region where polystyrene particles are condensed in contact arrangement is a region distant from fiber side surface 502 by a distance approximately as large as the diameter (125 $\mu$m in this example) of optical fiber 50.

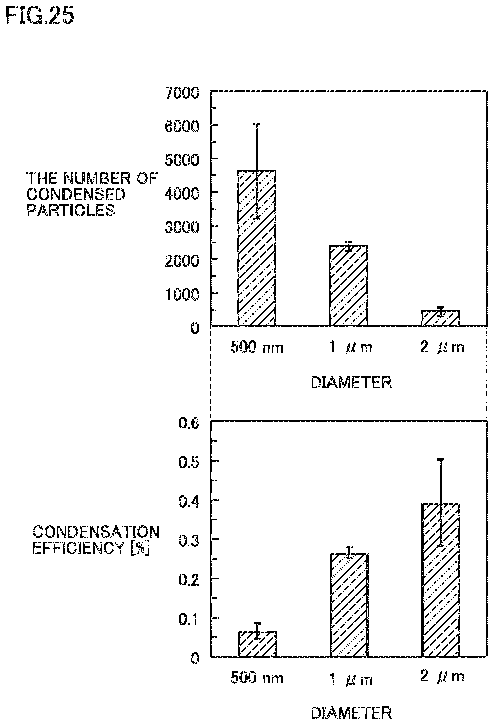

Fig. 25 is a diagram showing size dependency of the results of condensation of microscopic objects in the example where fiber end 501 is arranged at the bottom portion of the sample (contact arrangement). The abscissa represents the diameter of polystyrene particles. The upper ordinate represents the number of condensed polystyrene particles and the lower ordinate represents efficiency in condensation of polystyrene particles.

The concentration of polystyrene particles (YG) each having a diameter of 500 nm was $3.69\times10^8$ [particles/mL]. The concentration of polystyrene particles (YG) each having a diameter of 1 $\mu$m was $4.5\times510^7$ [particles/mL]. The concentration of polystyrene particles (YG) each having a diameter of 2 $\mu$m was $4.5\times510^7$ [particles/mL]. A volume ratio of one polystyrene particle among these three types of samples is calculated as 500 $\mu$m: 1 $\mu$m: 2 $\mu$m = 0.125: 1: 8. A concentration ratio of polystyrene particles in the sample, on the other hand, is calculated as 500 $\mu$m: 1 $\mu$m: 2 $\mu$m = 8.01: 1: 0.081. Therefore, the three types of samples were substantially equal to one another in volume ratio of all polystyrene particles occupied in the sample.

As the diameter of polystyrene particles is smaller, the number of condensed particles is larger whereas condensation efficiency was lower, which indicates that, when the volume ratio of all polystyrene particles occupied in the sample is equal, as the diameter of polystyrene particles is larger, condensation efficiency is higher. In other words, particles having a large size can efficiently be condensed.

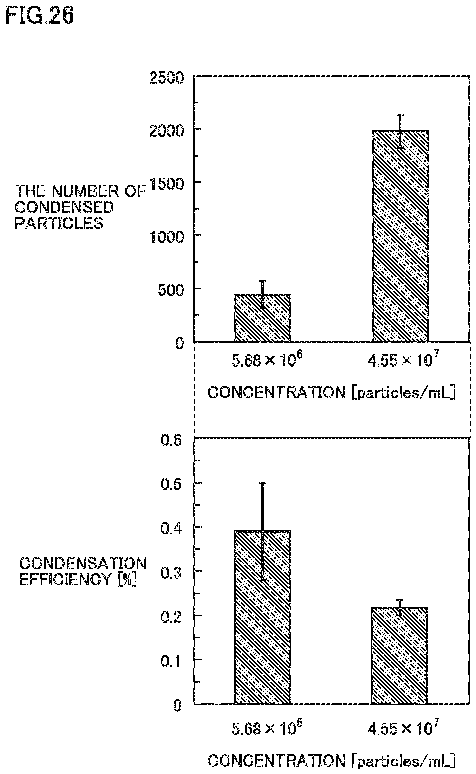

Fig. 26 is a diagram showing concentration dependency of the results of condensation of microscopic objects in the example where fiber end 501 is arranged at the bottom portion of the sample (contact arrangement). The abscissa represents the concentration of polystyrene particles. The upper ordinate represents the number of condensed polystyrene particles and the lower ordinate represents efficiency in condensation of polystyrene particles.

Polystyrene particles (YG) each having a diameter of 2 $\mu$m were employed. The concentration of polystyrene particles in one of the two samples was $5.68 \times 10^6$ [particles/mL]. The concentration of polystyrene particles in another sample was $4.5 \times 510^7$ [particles/mL],

As the concentration of polystyrene particles was lower, condensation efficiency was higher. This may be because, at the high concentration, the collection site around microbubble is saturated by collected polystyrene particles, which makes further collection difficult.

«Flow-Field Analysis»

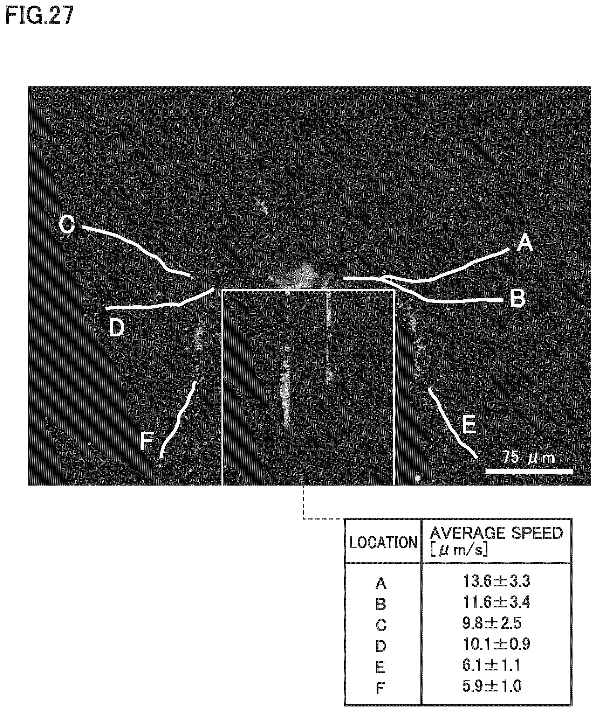

Fig. 27 is a diagram showing a result of analysis of how heat convection flows in the example where fiber end 501 is arranged at the bottom portion of the sample (contact arrangement). The sample containing polystyrene particles at the concentration of $5.68 \times 10^6$ [particles/mL], the polystyrene particle having a diameter of 2 $\mu$m and being high in condensation efficiency in Fig. 26, was employed. Flow-field analysis by particle tracking was conducted to obtain the flow velocity of heat convection at six locations shown in Fig. 27.

The flow velocities (5 and 6) around fiber side surface 502 were lower than the flow velocities (1 to 4) in the vicinity of microbubble (fiber end 501). This is one of pieces of evidence showing that the stagnation region is produced on fiber side surface 502 to become the collection site in contact arrangement. Not only heat convection but also a capillary phenomenon that occurs between optical fiber 50 and condensation kit 11 (cover glass in this example) and/or thermophoresis caused by a temperature gradient that occurs at fiber end 501 may contribute to drive force to condense polystyrene particles around fiber side surface 502.

[Sixth Example]

<Change in Characteristic of Metallic Thin Film>

Influence by change in characteristic of the metallic thin film formed at fiber end 501 will be described.

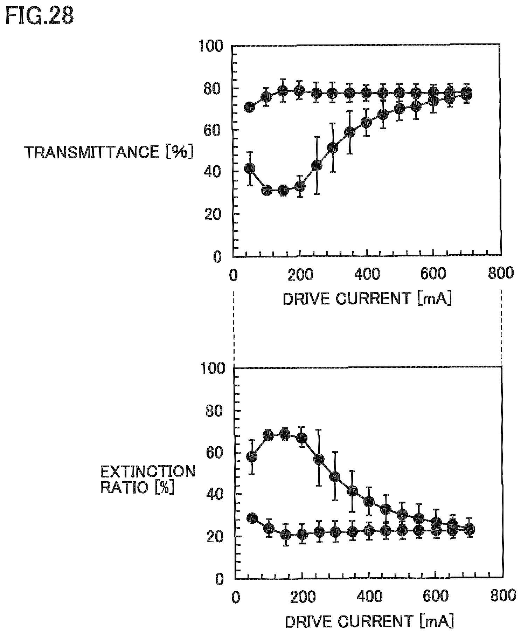

Fig. 28 is a diagram showing a transmittance ratio and an extinction ratio of laser beams when the drive current of laser light source 40 is varied. The abscissa represents the drive current of laser light source 40. The ordinate in the upper figure represents the transmittance ratio at fiber end 501. The ordinate in the lower figure represents the extinction ratio at fiber end 501. The extinction ratio satisfies a relational expression of the extinction ratio = absorptance + reflectance = 100% - transmittance ratio.

In measurement shown in Fig. 28, the drive current was increased from 50 mA to 700 mA in increments of 50 mA, and thereafter the drive current was lowered from 700 mA to 50 mA in decrements of 50 mA. A time interval of variation in drive current was set to ten seconds. Consequently, both of the transmittance ratio and the extinction ratio were different between a case of increase in drive current and a case of subsequent lowering in drive current. In other words, there was a hysteresis in transmittance ratio and extinction ratio. In particular, when the drive current was lowered after the drive current was increased, the extinction ratio at fiber end 501 became constant at approximately 20%.

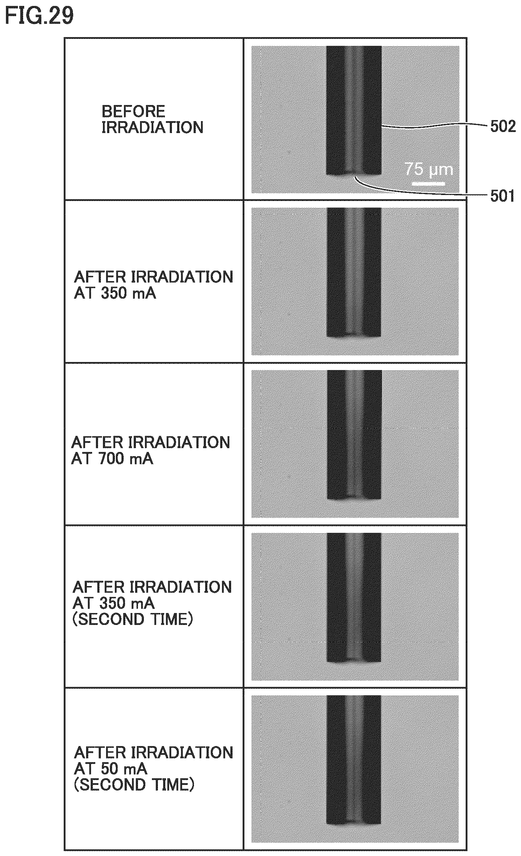

Fig. 29 is a diagram showing an image obtained by imaging of fiber end 501 before and after variation in drive current of laser light source 40. Fiber end 501 has a flat end surface. Though it is relatively unintelligible in a monochrome image, while the drive current was sequentially increased, color change of fiber end 501 was observed when the drive current was within a range from 150 mA to 350 mA. Color change of fiber end 501 was observed over a wide range including fiber side surface 502 in addition to fiber end 501, which suggests possibility of change in characteristic of metallic thin film 52 (the gold thin film in this example) over the wide range. Though a mechanism of change in characteristic of metallic thin film 52 is unclear at the current stage, influence on the photothermal effect may be possible.

According to the results shown in Figs. 28 and 29, as pre-treatment for the condensation step (processing in S9 in Fig. 4), processing for increasing the drive current can be added prior to lowering the drive current. Change in characteristic of optical fiber 50 (fiber end 501 and fiber side surface 502) can thus positively be caused. By causing change in characteristic of optical fiber 50 in advance to stabilize the characteristic, change in characteristic that may be caused by variation in drive current during the condensation step (that is, variation in output of light that propagates through optical fiber 50) can be suppressed. An optical condition for condensation of microscopic objects can thus be fixed. Therefore, for example, in performing the condensation step on a plurality of samples, conditions can be unified among the samples.

When microbubble grows at the bottom surface of the container, the main surface of the substrate, or the like as in PTLs 1 and 2, the size of microbubble may be varied for each time of measurement. In contrast, when microbubble grows at fiber end 501, the size of microbubble may be dependent on the diameter of optical fiber 50 (an area of fiber end 501). Since the diameter of optical fiber 50 has a fixed value (125 $\mu$m in this example) in accordance with specifications of optical fiber 50, variation in size of microbubble can be reduced by employing optical fiber 50. This aspect also serves to unify conditions among a plurality of samples in performing the condensation step on the samples.

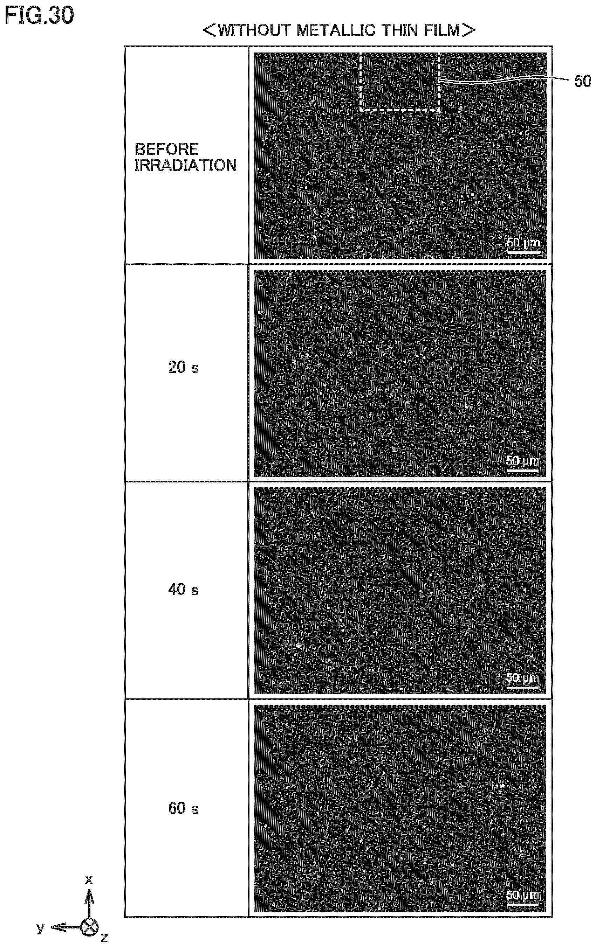

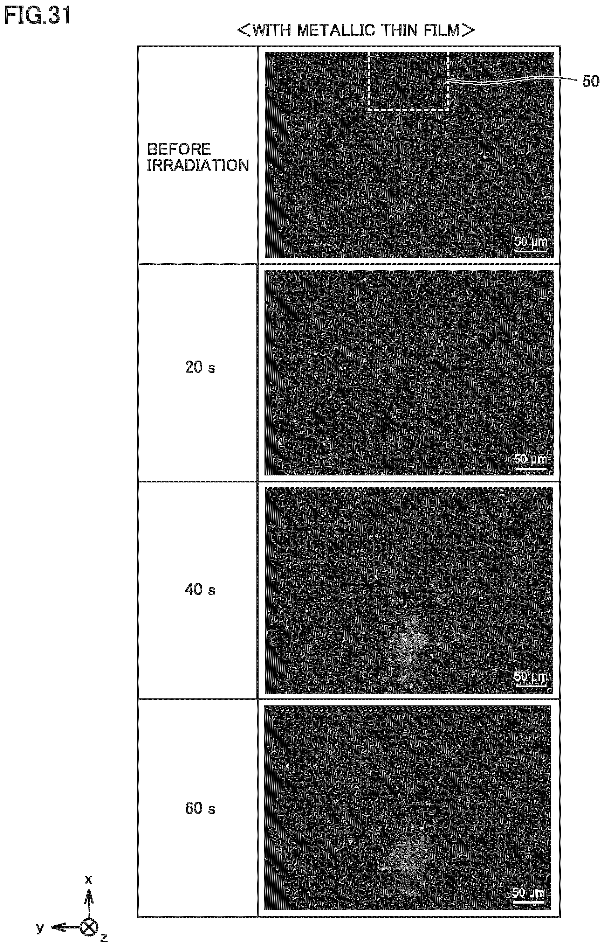

Fig. 30 is a diagram showing successive images of the results of condensation of microscopic objects in the example where metallic thin film 52 is not formed at fiber end 501. Fig. 31 is a diagram showing successive images of the results of condensation of microscopic objects in the example where metallic thin film 52 is formed at fiber end 501. Fig. 31 shows the results of condensation after addition of processing for causing change in characteristic of metallic thin film 52.

Polystyrene particles each having a diameter of 1 $\mu$m were employed as microscopic objects. The concentration of polystyrene particles was $4.5 \times 510^7$ [particles/mL]. The volume of the sample was set to 20 $\mu$L. The drive current was set to 700 mA. The time period for irradiation with laser beams was set to sixty seconds. Tween®20 was employed as the surfactant. The concentration of the surfactant was $5.43 \times 10^{-5}$ M.

In the example where metallic thin film 52 was not formed, polystyrene particles merely moved at a low speed owing to light-induced force produced with irradiation with laser beams, and heat convection was not produced. Therefore, optical condensation of polystyrene particles did not occur (see Fig. 30). In the example where metallic thin film 52 was formed, on the other hand, polystyrene particles were carried at a high speed toward fiber end 501 owing to light-induced force (more specifically, dissipative force) and heat convection, and condensed in front of fiber end 501 (see Fig. 31), although no microbubble was produced.

Production of no microbubble in measurement shown in Fig. 31 may be because of relatively small temperature increase with irradiation with laser beams. By performing the condensation step under such a condition, while thermal damage which may be caused in microscopic objects and condensation kit 11 (metallic thin film 52 or the like) is suppressed, microscopic objects can highly efficiently be collected and condensed. This effect is a particularly important advantage in an example where microscopic objects are heat-sensitive like bacteria.

[Seventh Example]

<Microscopic Objects that Can Optically Be Condensed>

A result of optical condensation of various microscopic objects other than polystyrene particles will be described.

<<Bacteria>>

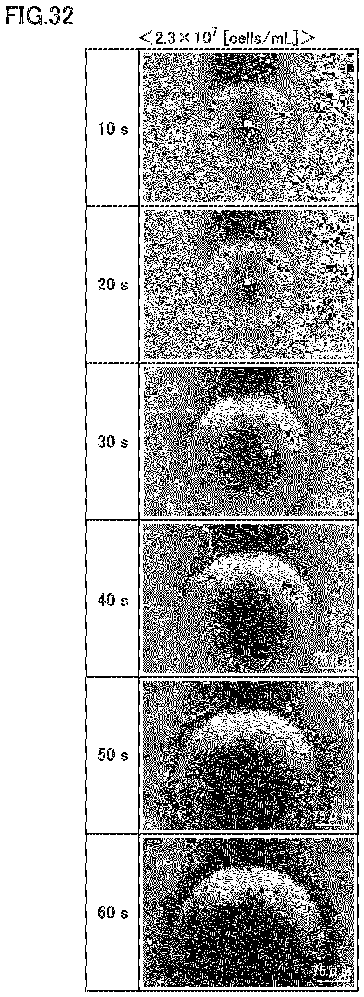

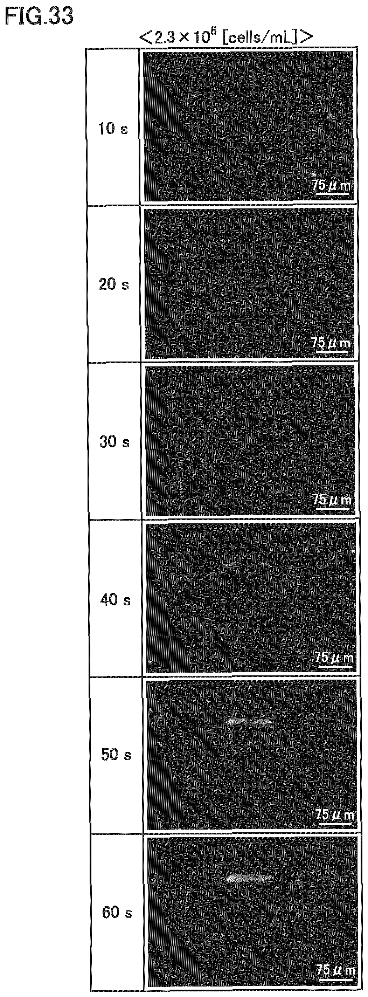

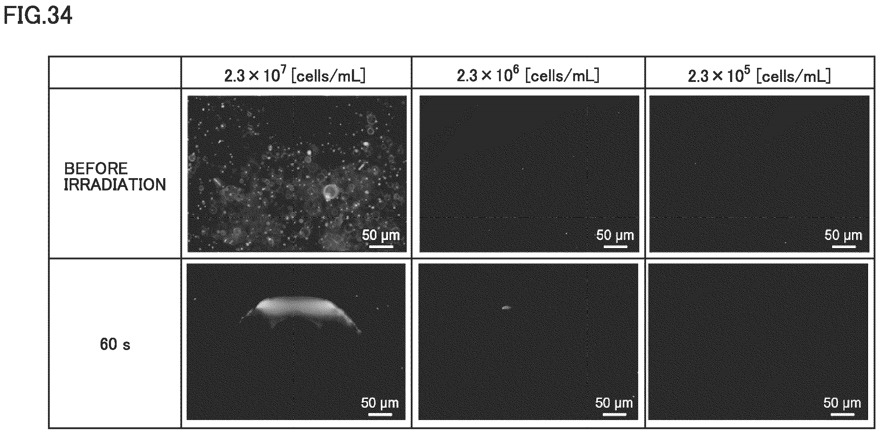

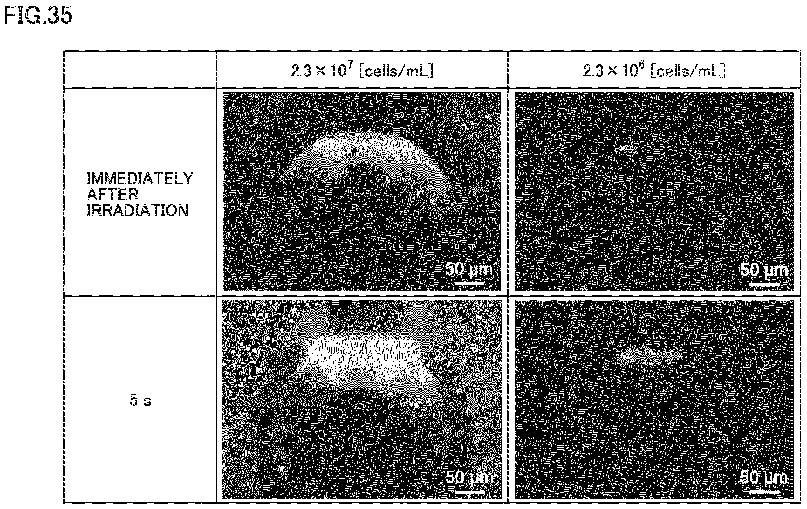

Figs. 32 and 33 are each a diagram where fluorescent observation images of results of condensation of bacteria are chronologically aligned. Figs. 32 and 33 show the results of condensation of bacteria when concentrations of bacteria are different from each other. Fig. 34 is a diagram for comparison of the fluorescent observation images of the results of condensation of bacteria between before and after irradiation with laser beams. Fig. 35 is a diagram showing results of observation of bacteria after irradiation with laser beams is stopped. Fig. 35 shows a state of bacteria immediately after stop of irradiation with laser beams and a state five seconds thereafter.

*Escherichia coli* (*Escherichia coli or E. coli*) was employed as bacteria. A concentration of bacteria was set to three patterns of $2.3 \times 10^5$, $2.3 \times 10^6$, and $2.3 \times 10^7$ [cells/mL]. SYTO®9 was employed as a fluorescent dye. SYTO®9 dyes both of living bacteria and dead bacteria. The drive current of laser light source 40 was set to 700 mA (laser output of 401 mW).

As shown in Figs. 32 to 34, when the concentration of bacteria was set to $2.3 \times 10^6$ or $2.3 \times 10^7$ [cells/mL], optical condensation of bacteria around fiber end 501 (including a portion in front of fiber end 501) could be confirmed. When the concentration of bacteria was set to $2.3 \times 10^7$ [cells/mL], optical condensation of bacteria was particularly significantly observed. As shown in Fig. 35, when irradiation with laser beams is stopped, diffusion of bacteria condensed at fiber end 501 into the sample was observed.

<<Nanodiamond>>

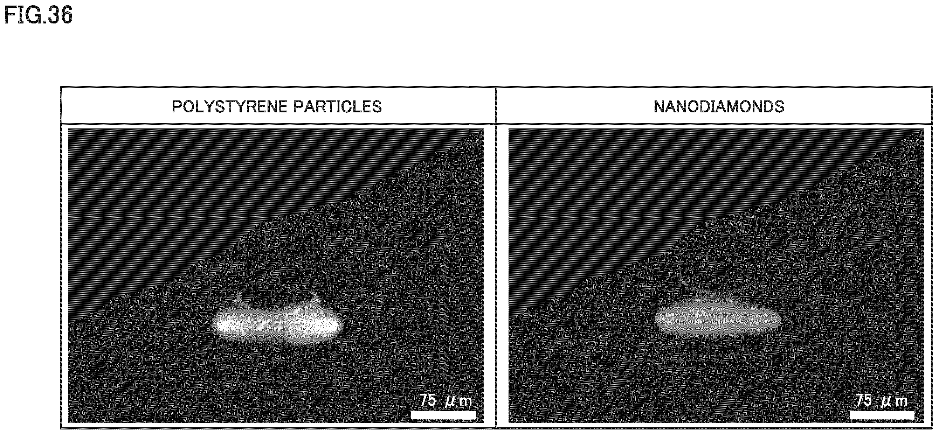

Fig. 36 is a diagram showing a fluorescent observation image of a result of condensation of nanodiamond. Nanodiamond is one type of a quantum sensor (quantum probe) which is substantially fade-free and important in cell imaging and assessment of characteristics (a temperature, pH, or the like) in the inside of cells. The quantum sensor is a sensor for measurement of a trace physical amount by using laws of quantum mechanics. The quantum sensor is expected as a next-generation supersensitive sensing technique not only in life science but also in various fields such as the IoT, environments, and energy. Fig. 36 shows also a result of condensation of polystyrene particles for the purpose of comparison.

A concentration of nanodiamond having a diameter of 100 nm was $5.43 \times 10^{10}$ [particles/mL]. The concentration of polystyrene particles each having a diameter of 100 nm was $4.55 \times 10^{10}$ [particles/mL] which was also approximately the same. In contactless arrangement, laser output was set to 320 mW.

The number of condensed nanodiamonds was $(2.5\pm1.4)\times10^7$ [particles], which was larger than the number of condensed polystyrene particles of $(8.2\pm4.6)\times10^6$ [particles]. Efficiency in condensation of nanodiamond was $2.3\pm1.3\%$, which was higher than efficiency in condensation of polystyrene particles of $0.89\pm0.5\%$.

Thus, it was demonstrated that not only optical condensation of biological substances such as bacteria and cells but also optical condensation of nanodiamond to be used for cell imaging or the like could be achieved. The quantum sensor may include a fluorescent molecule, a quantum dot, a metallic nanoparticle, a metallic nanorod, and the like other than nanodiamond. These quantum sensors (one type or a plurality of types being applicable) can also optically be condensed similarly to nanodiamond.

[Eighth Example]

<Long-Distance Optical Condensation>

A third manner of optical condensation different from optical condensation in contactless arrangement or contact arrangement described in the fifth example will be described.

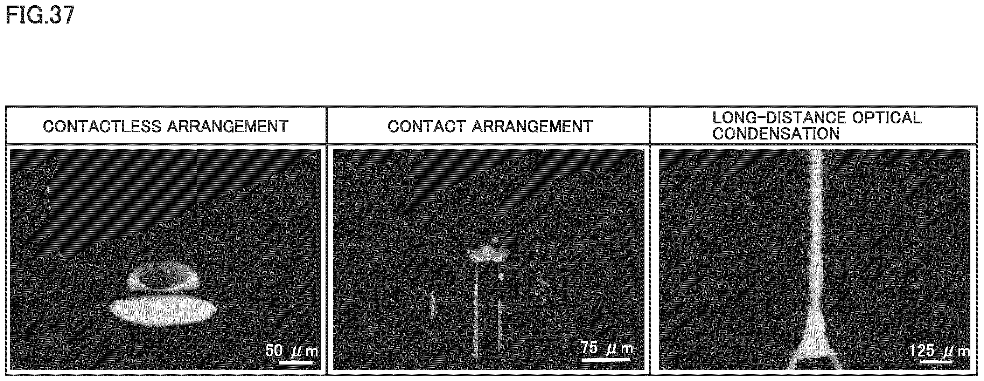

Fig. 37 is a diagram where three manners of optical condensation are organized. In contact arrangement of the optical fiber provided with the tip end (flat end surface) changed in characteristic (stabilized in characteristic) described in the sixth example, as shown in the right figure in Fig. 37, polystyrene particles were optically condensed over a long distance along an optical path of laser beams emitted from the tip end of the optical fiber. This manner is referred to as "long-distance optical condensation." In this example, the distance over which long-distance optical condensation occurs is longer than a range of imaging of 0.85 mm in the vertical direction in the right figure.

A result of optical condensation in contactless arrangement is obtained by preparation and use of polystyrene particles at the concentration of $4.55\times10^7$ [particles/mL], each having a diameter of 1 $\mu$m. Optical condensation in contact arrangement is achieved by preparation and use of polystyrene particles at the concentration of $5.68\times10^6$ [particles/mL], each having a diameter of 2 $\mu$m. Long-distance optical condensation is achieved by preparation and use of polystyrene particles at the concentration of $4.5\times510^7$ [particles/mL], each having a diameter of 2 $\mu$m.

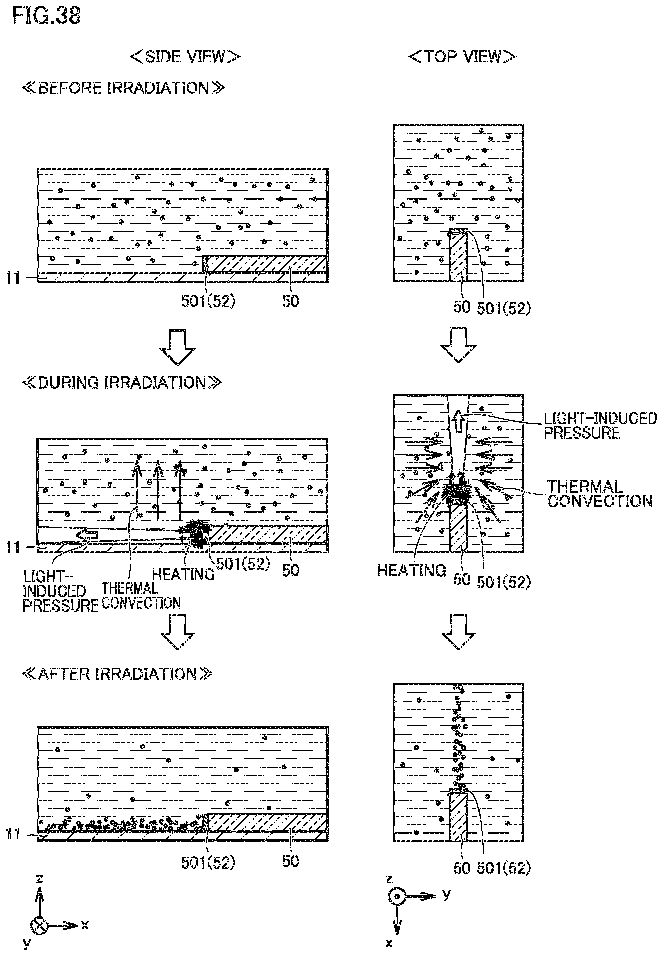

Fig. 38 is a diagram for illustrating a mechanism of long-distance optical condensation. Chronological side views are shown on the left side, and chronological top views are shown on the right side.

With irradiation with laser beams, the vicinity of fiber end 501 is locally heated owing to the photothermal effect of metallic thin film 52 provided at fiber end 501. Heat convection is thus produced. In addition, as light-induced force originating from laser beams that has passed through metallic thin film 52 is applied to microscopic objects, microscopic objects are carried. Furthermore, evanescent waves may be induced at the surface of condensation kit 11 (the surface of cover glass included in condensation kit 11) to produce light-induced force. Thus, carry of microscopic objects by both of heat convection and light-induced force may contribute to long-distance optical condensation. Light-induced force may be one or both of light-induced force originating from laser beams that passes through metallic thin film 52 and light-induced force originating from evanescent waves induced at the surface of condensation kit 11. Alternatively, microscopic objects can also be carried only by light-induced force originating from laser beams that passes through metallic thin film 52 and/or light-induced force originating from evanescent waves induced at the surface of condensation kit 11, without production of heat convection.

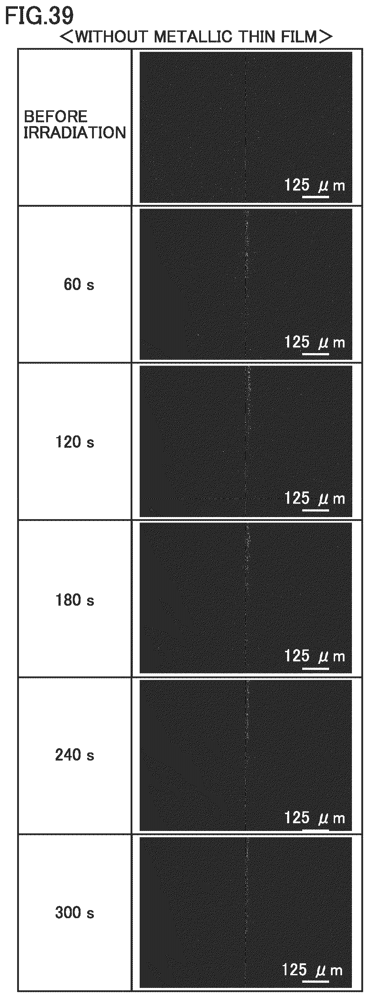

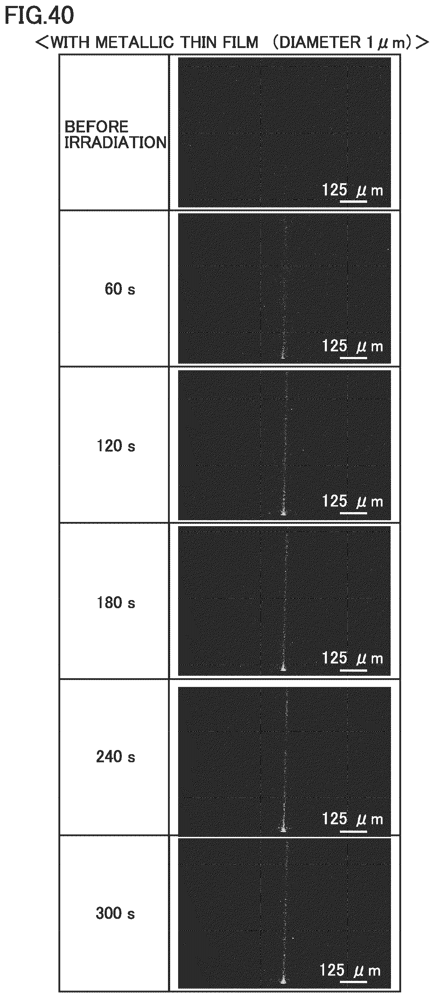

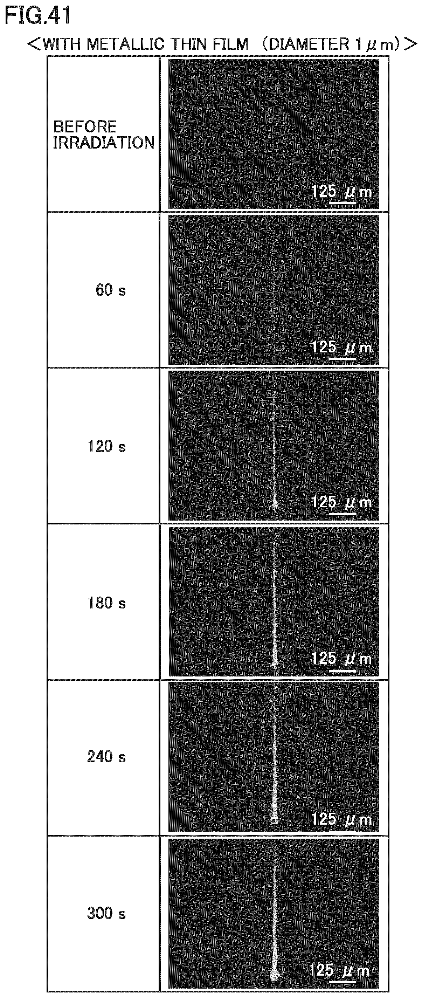

Fig. 39 is a diagram where fluorescent observation images of results of optical condensation in a comparative example are chronologically aligned. Figs. 40 and 41 are each a diagram where fluorescent observation images of results of optical condensation in the present example are chronologically aligned.

Fig. 39 shows results in the example where metallic thin film 52 is not provided. Figs. 40 and 41 show results in the example where the metallic thin film (having a thickness of 10 nm) is provided at the tip end of the optical fiber and characteristics of the metallic thin film have changed. The size (the diameter of 1 $\mu$m) and the concentration ($4.55\times10^7$ [particles/mL]) of polystyrene particles are equal between Figs. 39 and 40. In Fig. 41, the concentration of polystyrene particles ($4.55\times10^7$ [particles/mL]) is equal whereas the size (the diameter of 2 $\mu$m) of polystyrene particles is different. In any case, irradiation with laser at 540 mW for one minute in air was performed, and thereafter irradiation with laser at 485 mW for five minutes in the sample was performed. Laser beams are emitted upward from below in the figures.

In Fig. 39 showing the comparative example, optical condensation did not occur at fiber end 501 (a lower portion in each image) but optical condensation occurred at a position distant from the tip end. In contrast, in Fig. 40 showing the present example, optical condensation starting from fiber end 501 was highly efficiently observed over a long distance. In Fig. 41 where the diameter of polystyrene particles was large, optical condensation over a longer distance more highly efficient than in Fig. 40 was observed.

As set forth above, also in the second embodiment as in the first embodiment, microscopic objects are condensed in the vicinity of the tip end of optical fiber 50 by making use of the photothermal effect of metallic thin film 52 formed at the tip end of optical fiber 50. The tip end of optical fiber 50 can be adjusted to any position in the sample. Therefore, according to the second embodiment, microscopic objects can be condensed at any location in the sample.

The configuration in which the tip end of optical fiber 50 is arranged in the sample (including the gas-liquid interface) is described in the first and second embodiments. In production of heat convection, however, arrangement of the tip end of optical fiber 50 in the sample is not essential, and the tip end of optical fiber 50 may be arranged outside the sample. This is because, even when the tip end of optical fiber 50 is arranged outside the sample, a temperature distribution can be generated in the sample owing to the photothermal effect, so long as the distance between optical fiber 50 and the sample is not excessively long. The tip end of optical fiber 50 is arranged at a position where convection is produced in the sample by heating of the sample in introduction into optical fiber 50, of light at the wavelength included in the range of the absorption wavelength of metallic thin film 52.

The first to fourth examples are described in the first embodiment and the fifth to eighth examples are described in the second embodiment. These examples can be combined as appropriate. For example, contact arrangement/contactless arrangement (the fifth example) can naturally be applied to each of the first to fourth examples in the first embodiment. Change in characteristic (the sixth example) of the metallic thin film can be applied to the first to fourth examples in the first embodiment and also to other fifth and seventh examples in the second embodiment. The surfactant (the third example) can also be applied to the first, second, and fourth examples in the first embodiment and the fifth to eighth examples in the second embodiment.

[Aspects]

Illustrative embodiments described above are understood by a person skilled in the art as specific examples of aspects below.

<Clause 1>

A microscopic object condensation method including:

preparing an optical fiber including a tip end provided with a photothermal conversion material;
arranging the tip end in liquid where a plurality of microscopic objects are dispersed; and
introducing light into the optical fiber to heat the liquid around the tip end thereby producing convection, the light having a wavelength included in a range of an absorption wavelength of the photothermal conversion material.

<Clause 2>

The microscopic object condensation method according to Clause 1, in which
the arranging the tip end includes adjusting a position or a height of the tip end in the liquid.

<Clause 3>

The microscopic object condensation method according to Clause 1 or 2, in which
the preparing the optical fiber includes pre-treating the photothermal conversion material to stabilize a transmittance ratio or an extinction ratio of the photothermal conversion material with respect to variation in output of light that propagates through the optical fiber.

<Clause 4>

The microscopic object condensation method according to any one of Clauses 1 to 3, in which
the producing convection includes generating a microbubble at the tip end and condensing the plurality of microscopic objects in a region between the tip end and the microbubble.

<Clause 5>

The microscopic object condensation method according to Clause 3, in which

the arranging the tip end includes setting arrangement of the optical fiber with respect to a substrate where the liquid is held to one of contactless arrangement and contact arrangement,
the contactless arrangement refers to arrangement where a propagation path of the optical fiber is not in contact with the substrate, and
the contact arrangement refers to arrangement where the propagation path of the optical fiber is in contact with the substrate.

<Clause 6>

The microscopic object condensation method according to Clause 5, in which

the arranging the tip end is setting the optical fiber in the contactless arrangement, and
the producing convection includes condensing the plurality of microscopic objects at the tip end without generation of a microbubble at the pre-treated tip end.

<Clause 7>

The microscopic object condensation method according to Clause 5, in which

the arranging the tip end is setting the optical fiber in the contact arrangement, and
the producing convection includes condensing the plurality of microscopic objects along an optical path of light emitted from the tip end.

<Clause 8>

The microscopic object condensation method according to any one of Clauses 1 to 7, further including introducing a surfactant into the liquid prior to the producing convection.

<Clause 9>

The microscopic object condensation method according to Clause 8, in which
the introducing the surfactant includes preparing a concentration of the surfactant in the liquid to a critical micelle concentration.

<Clause 10>

The microscopic object condensation method according to any one of Clauses 1 to 9, in which

each of the plurality of microscopic objects is a quantum sensor, and
the quantum sensor includes at least one of nanodiamond, a fluorescent molecule, a quantum dot, a metallic nanoparticle, and a metallic nanorod.

<Clause 11>

A microscopic object condensation method of condensing a plurality of microscopic objects dispersed in liquid, the microscopic object condensation method including:

preparing an optical fiber including a tip end provided with a photothermal conversion material; and
arranging the tip end at a position where convection is produced in the liquid as a result of introduction of light into the optical fiber to heat the liquid, the light having a wavelength included in a range of an absorption wavelength of the photothermal conversion material.

<Clause 12>

The microscopic object condensation method according to claim 11, in which

the arranging the tip end includes arranging the tip end at a position where light-induced force in addition to the convection is produced in the liquid, and
the light-induced force includes at least one of light-induced force originating from light that passes through the photothermal conversion material and light-induced force originating from evanescent waves induced by light that propagates through the optical fiber at a surface of a substrate where the liquid is held.

<Clause 13>

A microscopic object condensation method of condensing a plurality of microscopic objects dispersed in liquid, the microscopic object condensation method including:

preparing an optical fiber including a tip end; and
arranging the tip end at a position where light-induced force is produced in the liquid in introduction of light into the optical fiber, in which
the light-induced force includes at least one of light-induced force originating from light emitted from the tip end and light-induced force originating from evanescent waves induced by light that propagates through the optical fiber at a surface of a substrate where the liquid is held.

<Clause 14>

A microscopic object condensation kit including:

a substrate configured to hold on a main surface, liquid where a plurality of microscopic objects are dispersed; and
an optical fiber including a tip end provided with a photothermal conversion material, in which
the optical fiber is configured such that the tip end is arranged in the liquid when the liquid is held on the main surface.

<Clause 15>

The microscopic object condensation kit according to Clause 14, in which
the photothermal conversion material has a color changed by variation in output of light that propagates through the optical fiber.

<Clause 16>

The microscopic object condensation kit according to Clause 14 or 15, in which
the tip end has a shape of a perfect circle.

<Clause 17>

The microscopic object condensation kit according to any one of Clauses 14 to 16, in which
the optical fiber is a multi-mode fiber.

<Clause 18>

A microscopic object condensation system comprising:

an optical fiber including a first end provided with a photothermal conversion material and a second end;
an adjustment mechanism that adjusts a position or a height of the first end in liquid where a plurality of microscopic objects are dispersed while the liquid is held in a condensation kit; and
a light source optically coupled to the second end, the light source emitting light at a wavelength included in a range of an absorption wavelength of the photothermal conversion material.

<Clause 19>

The microscopic object condensation system according to Clause 18, in which

the light source causes generation of a microbubble at the first end by heating of the liquid around the first end with light,
the microscopic object condensation system includes

an imager that takes an image of a region between the first end and the microbubble, and
a processor that calculates the number of condensed microscopic objects in the region in accordance with an expression (1) below obtained from the image

[Expression 5]

$$N = \frac{\pi}{6}\text{h}\frac{2r_1^2+r_1r_2-h^2-3r_3^2-r_2^2}{V}\times F \quad \cdots (1)$$

where

N represents the number of condensed microscopic objects,
h represents a height of a condensation region where the plurality of microscopic objects are condensed,
$r_1$ represents a distance between a virtual central axis of the microbubble that extends perpendicularly to an end surface of the first end and an outer circumferential portion of the condensation region,
r2 represents a distance between the central axis and an inner circumferential portion of the condensation region,
r3 represents a distance between the central axis and a portion of the condensation region corresponding to the height,
V represents a volume of each of the plurality of microscopic objects, and
F represents a fill factor of a hexagonal close-packed structure.

<Clause 20>

The microscopic object condensation system according to Clause 18, in which

the light source causes generation of a microbubble at the first end by heating of the liquid around the first end with light,
the microscopic object condensation system includes

an imager that takes an image of a region between the first end and the microbubble, and
a processor that calculates the number of condensed microscopic objects in the region in accordance with an expression (2) below obtained from the image

[Expression 6]

$$N = \frac{\pi}{6}\text{h}\frac{-r_1^2+r_1r_2-h^2-3r_3^2+2r_2^2}{V}\times F \quad \cdots (2)$$

where

N represents the number of condensed microscopic objects,
h represents a height of a condensation region where the plurality of microscopic objects are condensed,
$r_1$ represents a distance between a virtual central axis of the microbubble that extends perpendicularly to an end surface of the first end and an outer circumferential portion of the condensation region,
r2 represents a distance between the central axis and an inner circumferential portion of the condensation region,
r3 represents a distance between the central axis and a portion of the condensation region corresponding to the height,
V represents a volume of each of the plurality of microscopic objects, and
F represents a fill factor of a hexagonal close-packed structure.

It should be understood that the embodiments disclosed herein are illustrative and non-restrictive in every respect. The scope of the present disclosure is defined by the terms of the claims rather than the description of the embodiments above and is intended to include any modifications within the scope and meaning equivalent to the terms of the claims.

REFERENCE SIGNS LIST

1 condensation system; 11, 12 condensation kit; 111 upper surface; 121 substrate; 122 cover; 123 spacer; 20 sample stage; 30 sample adjustment mechanism; 40 laser light source; 50 optical fiber; 51 propagation path; 52 metallic thin film; 501 fiber end; 502 fiber side surface; 60 fiber stage; 70 fiber adjustment mechanism; 81 illumination light source; 82 objective lens; 83 lens; 84 camera; 91 slide glass; 100 controller; 101 processor; 102 memory; 103 input and output port.

**Claims**

1. A microscopic object condensation method comprising:

   preparing an optical fiber including a tip end provided with a photothermal conversion material;
   arranging the tip end in liquid where a plurality of microscopic objects are dispersed; and
   introducing light into the optical fiber to heat the liquid around the tip end thereby producing convection, the light having a wavelength included in a range of an absorption wavelength of the photothermal conversion material.

2. The microscopic object condensation method according to claim 1, wherein
   the arranging the tip end includes adjusting a position or a height of the tip end in the liquid.

3. The microscopic object condensation method according to claim 1, wherein
   the preparing the optical fiber includes pre-treating the photothermal conversion material to stabilize a transmittance ratio or an extinction ratio of the photothermal conversion material with respect to variation in output of light that propagates through the optical fiber.

4. The microscopic object condensation method according to any one of claims 1 to 3, wherein
   the producing convection includes generating a microbubble at the tip end and condensing the plurality of microscopic objects in a region between the tip end and the microbubble.

5. The microscopic object condensation method according to claim 3, wherein

   the arranging the tip end includes setting arrangement of the optical fiber with respect to a substrate where the liquid is held to one of contactless arrangement and contact arrangement,
   the contactless arrangement refers to arrangement where a propagation path of the optical fiber is not in contact with the substrate, and
   the contact arrangement refers to arrangement where the propagation path of the optical fiber is in contact with the substrate.

6. The microscopic object condensation method according to claim 5, wherein

   the arranging the tip end is setting the optical fiber in the contactless arrangement, and
   the producing convection includes condensing the plurality of microscopic objects at the tip end without generation of a microbubble at the pre-treated tip end.

7. The microscopic object condensation method according to claim 5, wherein

   the arranging the tip end is setting the optical fiber in the contact arrangement, and
   the producing convection includes condensing the plurality of microscopic objects along an optical path of light emitted from the tip end.

8. The microscopic object condensation method according to any one of claims 1 to 3, further comprising introducing a surfactant into the liquid prior to the producing convection.

9. The microscopic object condensation method according to claim 8, wherein
   the introducing the surfactant includes preparing a concentration of the surfactant in the liquid to a critical micelle concentration.

10. The microscopic object condensation method according to any one of claims 1 to 3, wherein

    each of the plurality of microscopic objects is a quantum sensor, and
    the quantum sensor comprises at least one of nanodiamond, a fluorescent molecule, a quantum dot, a metallic nanoparticle, and a metallic nanorod.

11. A microscopic object condensation method of condensing a plurality of microscopic objects dispersed in liquid, the microscopic object condensation method comprising:

preparing an optical fiber including a tip end provided with a photothermal conversion material; and arranging the tip end at a position where convection is produced in the liquid as a result of introduction of light into the optical fiber to heat the liquid, the light having a wavelength included in a range of an absorption wavelength of the photothermal conversion material.

12. The microscopic object condensation method according to claim 11, wherein

the arranging the tip end includes arranging the tip end at a position where light-induced force in addition to the convection is produced in the liquid, and
the light-induced force includes at least one of light-induced force originating from light that passes through the photothermal conversion material and light-induced force originating from evanescent waves induced by light that propagates through the optical fiber at a surface of a substrate where the liquid is held.

13. A microscopic object condensation method of condensing a plurality of microscopic objects dispersed in liquid, the microscopic object condensation method comprising:

preparing an optical fiber including a tip end; and
arranging the tip end at a position where light-induced force is produced in the liquid in introduction of light into the optical fiber, wherein
the light-induced force includes at least one of light-induced force originating from light emitted from the tip end and light-induced force originating from evanescent waves induced by light that propagates through the optical fiber at a surface of a substrate where the liquid is held.

14. A microscopic object condensation kit comprising:

a substrate configured to hold on a main surface, liquid where a plurality of microscopic objects are dispersed; and
an optical fiber including a tip end provided with a photothermal conversion material, wherein
the optical fiber is configured such that the tip end is arranged in the liquid when the liquid is held on the main surface.

15. The microscopic object condensation kit according to claim 14, wherein
the photothermal conversion material has a color changed by variation in output of light that propagates through the optical fiber.

16. The microscopic object condensation kit according to claim 14 or 15, wherein
the tip end has a shape of a perfect circle.

17. The microscopic object condensation kit according to claim 14 or 15, wherein
the optical fiber is a multi-mode fiber.

18. A microscopic object condensation system comprising:

an optical fiber including a first end provided with a photothermal conversion material and a second end;
an adjustment mechanism that adjusts a position or a height of the first end in liquid where a plurality of microscopic objects are dispersed while the liquid is held in a condensation kit; and
a light source optically coupled to the second end, the light source emitting light at a wavelength included in a range of an absorption wavelength of the photothermal conversion material.

19. The microscopic object condensation system according to claim 18, wherein

the light source causes generation of a microbubble at the first end by heating of the liquid around the first end with light,
the microscopic object condensation system further comprises

an imager that takes an image of a region between the first end and the microbubble, and
a processor that calculates the number of condensed microscopic objects in the region in accordance with an expression (1) below obtained from the image [Expression 1]

$$N = \frac{\pi}{6}h\frac{2r_1^2 + r_1r_2 - h^2 - 3r_3^2 - r_2^2}{V} \times F \quad \cdots (1)$$

where

*N* represents the number of condensed microscopic objects,
h represents a height of a condensation region where the plurality of microscopic objects are condensed,
$r_1$ represents a distance between a virtual central axis of the microbubble that extends perpendicularly to an end surface of the first end and an outer circumferential portion of the condensation region,
$r_2$ represents a distance between the central axis and an inner circumferential portion of the condensation region,
$r_3$ represents a distance between the central axis and a portion of the condensation region corresponding to the height,
*V* represents a volume of each of the plurality of microscopic objects, and
F represents a fill factor of a hexagonal close-packed structure.

**20.** The microscopic object condensation system according to claim 18, wherein

the light source causes generation of a microbubble at the first end by heating of the liquid around the first end with light,
the microscopic object condensation system further comprises

an imager that takes an image of a region between the first end and the microbubble, and
a processor that calculates the number of condensed microscopic objects in the region in accordance with an expression (2) below obtained from the image [Expression 2]

$$N = \frac{\pi}{6}h\frac{-r_1^2 + r_1r_2 - h^2 - 3r_3^2 + 2r_2^2}{V} \times F \quad \cdots (2)$$

where

*N* represents the number of condensed microscopic objects,
*h* represents a height of a condensation region where the plurality of microscopic objects are condensed,
$r_1$ represents a distance between a virtual central axis of the microbubble that extends perpendicularly to an end surface of the first end and an outer circumferential portion of the condensation region,
$r_2$ represents a distance between the central axis and an inner circumferential portion of the condensation region,
$r_3$ represents a distance between the central axis and a portion of the condensation region corresponding to the height,
*V* represents a volume of each of the plurality of microscopic objects, and
F represents a fill factor of a hexagonal close-packed structure.

FIG.1

FIG.2

FIG.3

FIG.4

```
                    ┌─────────────┐
                    │    START    │
                    └─────────────┘
                           │
                           ▼                    ⌐S1
        ┌──────────────────────────────────────────┐
        │         PREPARE OPTICAL FIBER             │
        └──────────────────────────────────────────┘
                           │
                           ▼                    ⌐S2
        ┌──────────────────────────────────────────┐
        │  PREPARE SAMPLE IN WHICH MICROSCOPIC      │
        │  OBJECTS ARE DISPERSED                    │
        └──────────────────────────────────────────┘
                           │
                           ▼                    ⌐S3
        ┌──────────────────────────────────────────┐
        │          INTRODUCE SURFACTANT            │
        └──────────────────────────────────────────┘
                           │
                           ▼                    ⌐S4
        ┌──────────────────────────────────────────┐
        │  SET CONDENSATION KIT ON SAMPLE          │
        │  STAGE AND DROP SAMPLE                    │
        └──────────────────────────────────────────┘
                           │
                           ▼                    ⌐S5
        ┌──────────────────────────────────────────┐
        │         START IMAGING OF SAMPLE          │
        └──────────────────────────────────────────┘
                           │
                           ▼                    ⌐S6
        ┌──────────────────────────────────────────┐
        │  ADJUST POSITION AND HEIGHT OF           │
        │  SAMPLE STAGE                            │
        └──────────────────────────────────────────┘
                           │
                           ▼                    ⌐S7
        ┌──────────────────────────────────────────┐
        │  ADJUST POSITION AND HEIGHT OF FIBER     │
        │  END IN SAMPLE                           │
        └──────────────────────────────────────────┘
                           │
                           ▼                    ⌐S8
        ┌──────────────────────────────────────────┐
        │           EMIT LASER BEAMS               │
        └──────────────────────────────────────────┘
                           │
                           ▼                    ⌐S9
        ┌──────────────────────────────────────────┐
        │          CONDENSATION STEP               │
        └──────────────────────────────────────────┘
                           │
                           ▼                    ⌐S10
        ┌──────────────────────────────────────────┐
        │      STOP EMISSION OF LASER BEAMS        │
        └──────────────────────────────────────────┘
                           │
                           ▼                    ⌐S11
        ┌──────────────────────────────────────────┐
        │          QUIT IMAGING OF SAMPLE          │
        └──────────────────────────────────────────┘
                           │
                           ▼                    ⌐S12
        ┌──────────────────────────────────────────┐
        │  CALCULATE THE NUMBER OF CONDENSED       │
        │  MICROSCOPIC OBJECTS                     │
        └──────────────────────────────────────────┘
                           │
                           ▼
                    ┌─────────────┐
                    │     END     │
                    └─────────────┘
```

FIG.5

FIG.6

50

## FIG.7

(A)

(B)

(C)

(D)

FIG.8

EXTINCTION RATIO=
APPROXIMATELY 20%

450
400  · · · · · ·   WITHOUT METALLIC
              THIN FILM
350        ────   WITH METALLIC
300              THIN FILM
LASER   250
OUTPUT  200
[mW]    150
100
50
0

0    100   200   300   400   500   600   700   800

DRIVE CURRENT [mA]

EXTINCTION

FIG.9

# FIG.10

| WITHOUT SURFACTANT | WITH SURFACTANT |

125 μm     125 μm

z ⊗ → y
↓
x

FIG.11

FIG.12

# FIG.13

FIG.14

《CONTACTLESS ARRANGEMENT》

50

11

《CONTACT ARRANGEMENT》

50

11

z

x

y

FIG.15

# FIG.16

<700 mA (No. 3)>

# FIG.17

<600 mA (No. 5)>

# FIG.18

<500 mA (No. 6)>

**FIG.19**

| DRIVE CURRENT | 700 mA (No. 3) | 600 mA (No. 5) | 500 mA (No. 6) |
|---|---|---|---|
| IMAGE | 50 μm | | |
| THE NUMBER OF CONDENSED PARTICLES | 112753±41693 [particles] | 66051±19259 [particles] | 65192±15380 [particles] |
| CONDENSATION EFFICIENCY | 12.4±4.6 % | 7.3±2.1 % | 7.2±1.7 % |

z ⊗ → y
↓
x

EP 4 483 999 A1

FIG.20

| No. | DRIVE CURRENT [mA] | CONCENTRATION OF MICROSCOPIC OBJECTS [particles/mL] | CONCENTRATION OF SURFACTANT [M] | THE NUMBER OF CONDENSED PARTICLES [particles] | CONDENSATION EFFICIENCY [%] |
|---|---|---|---|---|---|
| 1 | 700 | $4.55 \times 10^7$ | 0 | N/A | N/A |
| 2 | 700 | $4.55 \times 10^7$ | $9.05 \times 10^{-6}$ | 110305±68871.8 | 12.1±7.6 |
| 3 | 700 | $4.55 \times 10^7$ | $5.43 \times 10^{-5}$ | 112753±41693 | 12.4±4.6 |
| 4 | 700 | $4.55 \times 10^7$ | $9.05 \times 10^{-5}$ | 104427±55599 | 11.5±6.1 |
| 5 | 600 | $4.55 \times 10^7$ | $5.43 \times 10^{-5}$ | 66051±19259 | 7.3±2.1 |
| 6 | 500 | $4.55 \times 10^7$ | $5.43 \times 10^{-5}$ | 65192±15380 | 7.2±1.7 |
| 7 | 500 | $4.55 \times 10^8$ | $5.43 \times 10^{-5}$ | 231168±37740 | 2.5±0.4 |
| 8 | 500 | $4.55 \times 10^6$ | $5.43 \times 10^{-5}$ | N/A | N/A |

FIG.21

EP 4 483 999 A1

<CONTACTLESS ARRANGEMENT>

BEFORE IRRADIATION

60 s

FIG.22

# FIG.23

| | BEFORE IRRADIATION | 60 s | 90 s |
|---|---|---|---|
| 700 mA | | | |
| 600 mA | | | |
| 500 mA | | | |

EP 4 483 999 A1

FIG.24        <CONTACT ARRANGEMENT>

| 1 $\mu$m<br>$4.55 \times 10^7$ [particles/mL] | |
| 500 nm<br>$3.64 \times 10^8$ [particles/mL] | |
| 2 $\mu$m<br>$4.55 \times 10^7$ [particles/mL] | |
| 2 $\mu$m<br>$5.69 \times 10^6$ [particles/mL] | |

FIG.25

FIG.26

FIG.27

| LOCATION | AVERAGE SPEED [$\mu$m/s] |
|----------|------------|
| A | 13.6±3.3 |
| B | 11.6±3.4 |
| C | 9.8±2.5 |
| D | 10.1±0.9 |
| E | 6.1±1.1 |
| F | 5.9±1.0 |

FIG.28

# FIG.29

| BEFORE IRRADIATION | |
| AFTER IRRADIATION AT 350 mA | |
| AFTER IRRADIATION AT 700 mA | |
| AFTER IRRADIATION AT 350 mA (SECOND TIME) | |
| AFTER IRRADIATION AT 50 mA (SECOND TIME) | |

FIG.30

<WITHOUT METALLIC THIN FILM>

FIG.31

<WITH METALLIC THIN FILM>

FIG.32

$<2.3 \times 10^7 \ [cells/mL]>$

FIG.33

$<2.3 \times 10^6$ [cells/mL]$>$

FIG.34

| | $2.3 \times 10^7$ [cells/mL] | $2.3 \times 10^6$ [cells/mL] | $2.3 \times 10^5$ [cells/mL] |
|---|---|---|---|
| BEFORE IRRADIATION | | | |
| 60 s | | | |

EP 4 483 999 A1

FIG.35

| | $2.3 \times 10^7$ [cells/mL] | $2.3 \times 10^6$ [cells/mL] |
|---|---|---|
| IMMEDIATELY AFTER IRRADIATION | 50 µm | 50 µm |
| 5 s | 50 µm | 50 µm |

FIG.36

| POLYSTYRENE PARTICLES | NANODIAMONDS |

75 μm   75 μm

FIG.37

| CONTACTLESS ARRANGEMENT | CONTACT ARRANGEMENT | LONG-DISTANCE OPTICAL CONDENSATION |
|---|---|---|
| 50 μm | 75 μm | 125 μm |

EP 4 483 999 A1

# FIG.38

<SIDE VIEW>                     <TOP VIEW>

≪BEFORE IRRADIATION≫

11

501(52)         50                    50 501(52)

≪DURING IRRADIATION≫

LIGHT-INDUCED
PRESSURE

THERMAL
CONVECTION

11

LIGHT-      THERMAL      HEATING  501(52)  50        HEATING  50 501(52)
INDUCED     CONVECTION
PRESSURE

≪AFTER IRRADIATION≫

11

501(52)         50                    50 501(52)

z                               z
↑                               ⊙ → y
⊗ → x                           ↓
y                               x

# FIG.39

<WITHOUT METALLIC THIN FILM>

| | |
|---|---|
| BEFORE IRRADIATION | 125 μm |
| 60 s | 125 μm |
| 120 s | 125 μm |
| 180 s | 125 μm |
| 240 s | 125 μm |
| 300 s | 125 μm |

# FIG.40

<WITH METALLIC THIN FILM (DIAMETER 1 $\mu$ m)>

## FIG.41

\<WITH METALLIC THIN FILM (DIAMETER 1 $\mu$ m)\>

## INTERNATIONAL SEARCH REPORT

| International application No. |
|---|
| **PCT/JP2023/006701** |

**A. CLASSIFICATION OF SUBJECT MATTER**

*B01J 19/00*(2006.01)i; *C12M 1/00*(2006.01)i; *C12M 1/42*(2006.01)i; *C12N 1/20*(2006.01)i
FI: B01J19/00 N; C12M1/00 A; C12M1/42; C12N1/20 Z

According to International Patent Classification (IPC) or to both national classification and IPC

**B. FIELDS SEARCHED**

Minimum documentation searched (classification system followed by classification symbols)

A61B18/20-18/28; B01J19/00-19/32; C12M1/00-1/42; C12N1/20-1/21; G01N27/00-27/92

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2018/207937 A1 (OSAKA PREFECTURE UNIVERSITY PUBLIC CORP.) 15 November 2018 (2018-11-15) claim 1, paragraphs [0001], [0009]-[0108], fig. 1, 2, 4, 5 | 1-20 |
| Y | JP 2013-141581 A (JEISYS JAPAN INC.) 22 July 2013 (2013-07-22) claims 1, 5, paragraphs [0001], [0007]-[0094] | 1-20 |
| Y | WO 2018/159706 A1 (OSAKA PREFECTURE UNIVERSITY PUBLIC CORP.) 07 September 2018 (2018-09-07) claims 19-22, paragraphs [0001], [0162]-[0169] | 8, 9 |
| Y | WO 2020/218347 A1 (UNIVERSITY PUBLIC CORP. OSAKA) 29 October 2020 (2020-10-29) paragraphs [0001], [0004]-[0101] | 19, 20 |

☐ Further documents are listed in the continuation of Box C.    ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **16 March 2023** | **04 April 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

International application No.

**PCT/JP2023/006701**

| Patent document cited in search report | | | Publication date (day/month/year) | Patent family member(s) | Publication date (day/month/year) |
|---|---|---|---|---|---|
| WO | 2018/207937 | A1 | 15 November 2018 | (Family: none) | |
| JP | 2013-141581 | A | 22 July 2013 | (Family: none) | |
| WO | 2018/159706 | A1 | 07 September 2018 | US 2019/0383708 A1 claims 19-22, paragraphs [0001], [0205]-[0213] | |
| WO | 2020/218347 | A1 | 29 October 2020 | (Family: none) | |

Form PCT/ISA/210 (patent family annex) (January 2015)

**REFERENCES CITED IN THE DESCRIPTION**

**Patent documents cited in the description**

- WO 2018159706 A

- WO 2020218347 A